(19) Europäisches Patentamt
European Patent Office
Office européen des brevets

(11) EP 0 955 374 B1

(12) EUROPÄISCHE PATENTSCHRIFT

(45) Veröffentlichungstag und Bekanntmachung des Hinweises auf die Patenterteilung:
**10.08.2005 Patentblatt 2005/32**

(51) Int Cl.[7]: **C12N 15/86**

(21) Anmeldenummer: **99250151.0**

(22) Anmeldetag: **07.05.1999**

# (54) Retrovirale Gentransfervektoren

Retroviral vectors for gene transfer

Vecteurs retroviraux pour le transfer de gènes

(84) Benannte Vertragsstaaten:
**DE FR GB IT SE**

(30) Priorität: **08.05.1998 DE 19822115**

(43) Veröffentlichungstag der Anmeldung:
**10.11.1999 Patentblatt 1999/45**

(73) Patentinhaber: **Heinrich-Pette-Institut**
**20251 Hamburg (DE)**

(72) Erfinder:
- **Ostertag, Wolfram, Prof., Dr.**
**22397 Hamburg (DE)**
- **Baum, Christopher, Dr.**
**22589 Hamburg (DE)**
- **Hildinger, Markus**
**20149 Hamburg (DE)**

(74) Vertreter: **UEXKÜLL & STOLBERG**
**Patentanwälte**
**Beselerstrasse 4**
**22607 Hamburg (DE)**

(56) Entgegenhaltungen:
**WO-A-96/07747** **WO-A-98/12338**

- **MILLER A D ET AL: "IMPROVED RETROVIRAL VECTORS FOR GENE TRANSFER AND EXPRESSION" BIOTECHNIQUES, NATICK, MA, US, Bd. 7, Nr. 9, 1. Oktober 1989 (1989-10-01), Seiten 980-990, XP000606889**
- **MILLER A D ET AL: "USE OF RETROVIRAL VECTORS FOR GENE TRANSFER AND EXPRESSION" METHODS IN ENZYMOLOGY, ACADEMIC PRESS INC, SAN DIEGO, CA, US, Bd. 217, 1993, Seiten 581-599, XP000916976 ISSN: 0076-6879**
- **DING S-F ET AL: "Co-packaging of sense and antisense RNAs: a novel strategy for blocking HIV-1 replication" NUCLEIC ACIDS RESEARCH, OXFORD UNIVERSITY PRESS, SURREY, GB, Bd. 26, Nr. 13, 1998, Seiten 3270-3278, XP002236827 ISSN: 0305-1048**
- **JOSHI S ET AL: "EFFICIENT REPLICATION, INTEGRATION, AND PACKAGING OF RETROVIRAL VECTORS WITH MODIFIED LONG TERMINAL REPEATS CONTAINING THE PACKAGING SIGNAL" NUCLEIC ACIDS RESEARCH, OXFORD UNIVERSITY PRESS, SURREY, GB, Bd. 18, Nr. 14, 1990, Seiten 4223-4226, XP001152991 ISSN: 0305-1048**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

SEQUENZPROTOKOLL

<110> Heinrich-Pette-Institut

<120> Retrovirale Gentransfervektoren

<130> P50490

<140>
<141>

<150> DE 198 22 115
<151> 1998-05-08

<160> 17

<170> PatentIn Vers. 2.0

<210> 1
<211> 8630
<212> DNA
<213> Künstliche Sequenz

<220>
<223> Beschreibung der künstlichen Sequenz: Provirale Plasmid-DNA

<220>
<221> misc_feature
<222> (1)..(160)
<223> Plasmid-Rückgrat (pUC)

<220>
<221> misc_feature
<222> (161)..(677)
<223> 5'-LTR

<220>
<221> 5'UTR
<222> (532)..(1219)

<220>
<221> mat_peptide
<222> (1220)..(5062)
<223> m4 mdr-1 cDNA

<220>
<221> misc_feature
<222> (5215)..(5774)
<223> 3'-LTR

<220>
<221> misc_feature
<222> (5775)..(8630)
<223> Plasmid-Rückgrat (pUC)

<220>

<221> misc_feature
<222> (1)..(8630)
<223> Retroviraler Expressionsvektor SFbeta71m4

<400> 1

```
tcgagggggg gcccggtcac gattagtcca atttgttaaa gacaggatat cagtggtcca 60
ggctctagtt ttgactcaac aatatcacca gctgaagcct atagagtacg agccatagat 120
agaataaaag attttattta gtctccagaa aaagggggga atgaaagacc ccacctgtag 180
gtttggcaag ctagcttaag taacgccatt ttgcaaggca tggaaaatac ataactgaga 240
atagagaagt tcagatcaag gttaggaaca gagagacagc agaatatggg ccaaacagga 300
tatctgtggt aagcagttcc tgccccgctc agggccaaga acagatggtc cccagatgcg 360
gtcccgccct cagcagtttc tagagaacca tcagatgttt ccagggtgcc ccaaggacct 420
gaaaatgacc ctgtgcctta tttgaactaa ccaatcagtt cgcttctcgc ttctgttcgc 480
gcgcttctgc tccccgagct caataaaaga gcccacaacc cctcactcgg cgcgccagtc 540
ctccgattga ctgagtcgcc cgggtacccg tattcccaat aaagcctctt gctgtttgca 600
tccgaatcgt ggactcgctg atccttggga gggtctcctc agattgattg actgcccacc 660
tcgggggtct ttcatttgga ggttccaccg agatttggag acccctgccc agggaccacc 720
gacccccccg ccgggaggta agctggccag cggtcgtttc gtgtctgtct ctgtctttgt 780
gcgtgtttgt gccggcatct aatgtttgcg cctgcgtctg tactagttgg ctaactagat 840
ctgtatctgg cggtcccgcg gaagaactga cgagttcgta ttcccggccg cagcccctgg 900
gagacgtccc agcggcctcg gggcccgtt ttgtggccca ttctgtatca gttaacctac 960
ccgagtcgga ctttttggag ctccgccact gtccgagggg tacgtggctt tgttgggga 1020
cgagagacag agacacttcc cgcccccgtc tgaatttttg ctttcggttt tacgccgaaa 1080
ccgcgccgcg cgtcttgtct gctgcagcat cgttctgtgt tgtctctgtc tgactgtgtt 1140
tctgtatttg tctgaaaatt agctcgacaa agttaactaa tagtccctct ctccaagctc 1200
acttacaggc gcggccgcca tggatcttga aggggaccgc aatggaggag caaagaagaa 1260
gaactttttt aaactgaaca ataaaagtga aaaagataag aaggaaaaga aaccaactgt 1320
cagtgtattt tcaatgtttc gctattcaaa ttggcttgac aagttgtata tggtggtggg 1380
aactttggct gccatcatcc atggggctgg acttcctctc atgatgctgg tgtttggaga 1440
aatgacagat atctttgcaa atgcaggaaa tttagaagat ctgatgtcaa acatcactaa 1500
tagaagtgat atcaatgata cagggttctt catgaatctg gaggaagaca tgaccagata 1560
tgcctattat tacagtggaa ttggtgctgg ggtgctggtt gctgcttaca ttcaggtttc 1620
attttggtgc ctggcagctg gaagacaaat acacaaaatt agaaaacagt tttttcatgc 1680
tataatgcga caggagatag gctggtttga tgtgcacgat gttggggagc ttaacacccg 1740
acttacagat gatgtctcta agattaatga agttattggt gacaaaattg gaatgttctt 1800
tcagtcaatg gcaacatttt tcactgggtt tatagtagga tttacacgtg gttggaagct 1860
aacccttgtg attttggcca tcagtcctgt tcttggactg tcagctgctg tctgggcaaa 1920
gatactatct tcatttactg ataaagaact cttagcgtat gcaaaagctg gagcagtagc 1980
tgaagaggtc ttggcagcaa ttagaactgt gattgcattt ggaggacaaa agaaagaact 2040
tgaaaggtac aacaaaaatt tagaagaagc taaaagaatt gggataaaga aagctattac 2100
agccaatatt tctataggtg ctgctttcct gctgatctat gcatcttatg ctctggcctt 2160
ctggtatggg accaccttgg tcctctcagg ggaatattct attggacaag tactcactgt 2220
attctttttct gtattaattg gggcttttag tgttggacag gcatctccaa gcattgaagc 2280
atttgcaaat gcaagaggag cagcttatga aatcttcaag ataattgata ataagccaag 2340
tattgacagc tattcgaaga gtgggcacaa accagataat attaagggaa atttggaatt 2400
cagaaatgtt cacttcagtt acccatctcg aaaagaagtt aagatcttga agggcctgaa 2460
cctgaaggtg cagagtgggc agacggtggc cctggttgga aacagtggct gtgggaagag 2520
cacaacagtc cagctgatgc agaggctcta tgaccccaca gaggggatgg tcagtgttga 2580
tggacaggat attaggacca taaatgtaag gtttctacgg gaaatcattg gtgtggtgag 2640
tcaggaacct gtattgtttg ccaccacgat agctgaaaac attcgctatg gccgtgaaaa 2700
tgtcaccatg gatgagattg agaaagctgt caaggaagcc aatgcctatg actttatcat 2760
gaaactgcct cataaatttg acaccctggt tggagagaga ggggcccagt tgagtggtgg 2820
gcagaagcag aggatcgcca ttgcacgtgc cctggttcgc aaccccaaga tcctcctgct 2880
ggatgaggcc acgtcagcct tggacacaga aagcgaagca gtggttcagg tggctctgga 2940
taaggccaga aaaggtcgga ccaccattgt gatagctcat cgtttgtcta cagttcgtaa 3000
tgctgacgtc atcgctggtt tcgatgatgg agtcattgtg gagaaaggaa atcatgatga 3060
actcatgaaa gagaaaggca tttacttcaa acttgtcaca atgcagacag caggaaatga 3120
agttgaatta gaaaatgcag ctgatgaatc caaaagtgaa attgatgcct tggaaatgtc 3180
ttcaaatgat tcaagatcca gtctaataag aaaaagatca actcgtagga gtgtccgtgg 3240
atcacaagcc caagacagaa agcttagtac caaagaggct ctggatgaaa gtatacctcc 3300
agtttccttt tggaggatta tgaagctaaa tttaactgaa tggccttatt ttgttgttgg 3360
tgtattttgt gccattataa atggaggcct gcaaccagca tttgcaataa tattttcaaa 3420
gattataggg gtttttacaa gaattgatga tcctgaaaca aaacgacaga atagtaactt 3480
gttttcacta ttgtttctag cccttggaat tatttctttt attacatttt tccttcaagg 3540
tttcacattt ggcaaagctg gagagatcct caccaagcgg ctccgataca tggttttccg 3600
atccatgctc agacaggatg tgagttggtt tgatgaccct aaaaacacca ctggagcatt 3660
gactaccagg ctcgccaatg atgctgctca agttaaaggg gctataggtt ccaggcttgc 3720
tgtaattacc cagaatatag caaatcttgg gacaggaata attatatcct tcatctatgg 3780
```

```
ttggcaacta acactgttac tcttagcaat tgtacccatc attgcaatag caggagttgt 3840
tgaaatgaaa atgttgtctg gacaagcact gaaagataag aaagaactag aaggtgctgg 3900
gaagatcgct actgaagcaa tagaaaactt ccgaaccgtt gtttctttga ctcaggagca 3960
gaagtttgaa catatgtatg ctcagagttt gcaggtacca tacagaaact ctttgaggaa 4020
agcacacatc tttggaatta cattttcctt cacccaggca atgatgtatt tttcctatgc 4080
tggatgtttc cggtttggag cctacttggt ggcacataaa ctcatgagct ttgaggatgt 4140
tctgttagta ttttcagctg ttgtctttgg tgccatggcc gtggggcaag tcagttcatt 4200
tgctcctgac tatgccaaag ccaaaatatc agcagcccac atcatcatga tcattgaaaa 4260
aacccctttg attgacagct acagcacgga aggcctaatg ccgaacacat tggaaggaaa 4320
tgtcacattt ggtgaagttg tattcaacta tcccacccga ccggacatcc cagtgcttca 4380
gggactgagc ctggaggtga agaagggcca gacgctggct ctggtgggca gcagtggctg 4440
tgggaagagc acagtggtcc agctcctgga gcggttctac gaccccttgg cagggaaagt 4500
gctgcttgat ggcaaagaaa ttaagcgact gaatgttcag tggctccgag cacacctggg 4560
catcgtgtcc caggagccca tcctgtttga ctgcagcatt gctgagaaca ttgcctatgg 4620
agacaacagc cgggtggtgt cacaggaaga gatcgtgagg gcagcaaagg aggccaacat 4680
acatgccttc atcgagtcac tgcctaataa atatagcact aaagtaggag acaaaggaac 4740
tcagctctct ggtggccaga aacaacgcat tgccatagct cgtgcccttg ttagacagcc 4800
tcatattttg cttttggatg aagccacgtc agctctggat acagaaagtg aaaaggttgt 4860
ccaagaagcc ctggacaaag ccagagaagg ccgcacctgc attgtgattg ctcaccgcct 4920
gtccaccatc cagaatgcag acttaatagt ggtgtttcag aatggcagag tcaaggagca 4980
tggcacgcat cagcagctgc tggcacagaa aggcatctat ttttcaatgg tcagtgtcca 5040
ggctggaaca aagcgccagt gaactctgac tgtatgagat gttaaatact ttttaatggg 5100
gatccaagct tatcgatagg cctaggccta tcgataggcc taggcctatc gataggccta 5160
acacgagcca tagatagaat aaaagatttt atttagtctc cagaaaaagg ggggaatgaa 5220
agaccccacc tgtaggtttg gcaagctagc tgcagtaacg ccattttgca aggcatggaa 5280
aaataccaaa ccaagaatag agaagttcag atcaagggcg ggtacatgaa aatagctaac 5340
gttgggccaa acaggatatc tgcggtgagc agtttcggcc ccggcccggg gccaagaaca 5400
gatggtcacc gcagtttcgg ccccggcccg aggccaagaa cagatggtcc ccagatatgg 5460
cccaaccctc agcagtttct taagacccat cagatgtttc caggctcccc caaggacctg 5520
aaatgaccct gcgccttatt tgaattaacc aatcagcctg cttctcgctt ctgttcgcgc 5580
gcttctgctt cccgagctct ataaaagagc tcacaacccc tcactcggcg cgccagtcct 5640
ccgattgact gagtcgcccg ggtacccgta ttcccaataa agcctcttgc tgtttgcatc 5700
cgaatcgtgg actcgctgat ccttgggagg gtctcctcag attgattgac tgcccacctc 5760
gggggtcttt cagtaggatc tcgaccgatg cccttgagag ccttcaaccc agtcagctcc 5820
ttccggtggg cgcggggcat gactatcgtc gccgcactta tgactgtctt ctttatcatg 5880
caactcgtag gacaggtgcc ggcagcgctc tgggtcattt tcggcgagga ccgctttcgc 5940
tggagcgcga cgatgatcgg cctgtcgctt gcggtattcg gaatcttgca cgccctcgct 6000
caagccttcg tcactggtcc cgccaccaaa cgtttcggcg agaagcaggc cattatcgcc 6060
ggcatggcgg ccgacgcgct gggctacgtc ttgctggcgt tcgcgacgcg aggctggatg 6120
gccttcccca ttatgattct tctcgcttcc ggcggcatcg ggatgcccgc gttgcaggcc 6180
atgctgtcca ggcaggtaga tgacgaccat cagggacagc ttcaaggatc gctcgcggct 6240
cttaccagcc taacttcgat cattggaccg ctgatcgtca cggcgattta tgccgcctcg 6300
gcgagcacat ggaacgggtt ggcatggatt gtaggcgcct gatgcggtat tttctcctta 6360
cgcatctgtg cggtatttca caccgcatat ggtgcactct cagtacaatc tgctctgatg 6420
ccgcatagtt aagccagccc cgacacccgc caacacccgc tgacgcgccc tgacgggctt 6480
gtctgctccc ggcatccgct tacagacaag ctgtgaccgt ctccgggagc tgcatgtgtc 6540
agaggttttc accgtcatca ccgaaacgcg cgagacgaaa gggcctcgtg atacgcctat 6600
ttttataggt taatgtcatg ataataatgg tttcttagac gtcaggtggc acttttcggg 6660
gaaatgtgcg cggaacccct atttgtttat ttttctaaat acattcaaat atgtatccgc 6720
tcatgagaca ataaccctga taaatgcttc aataatattg aaaaaggaag agtatgagta 6780
ttcaacattt ccgtgtcgcc cttattccct tttttgcggc attttgcctt cctgtttttg 6840
ctcacccaga aacgctggtg aaagtaaaag atgctgaaga tcagttgggt gcacgagtgg 6900
gttacatcga actggatctc aacagcggta agatccttga gagttttcgc cccgaagaac 6960
gttttccaat gatgagcact tttaaagttc tgctatgtgg cgcggtatta tcccgtattg 7020
acgccgggca agagcaactc ggtcgccgca tacactattc tcagaatgac ttggttgagt 7080
actcaccagt cacagaaaag catcttacgg atggcatgac agtaagagaa ttatgcagtg 7140
ctgccataac catgagtgat aacactgcgg ccaacttact tctgacaacg atcggaggac 7200
cgaaggagct aaccgctttt ttgcacaaca tgggggatca tgtaactcgc cttgatcgtt 7260
gggaaccgga gctgaatgaa gccataccaa acgacgagcg tgacaccacg atgcctgtag 7320
caatggcaac aacgttgcgc aaactattaa ctggcgaact acttactcta gcttcccggc 7380
aacaattaat agactggatg gaggcggata aagttgcagg accacttctg cgctcggccc 7440
ttccggctgg ctggtttatt gctgataaat ctggagccgg tgagcgtggg tctcgcggta 7500
tcattgcagc actggggcca gatggtaagc cctcccgtat cgtagttatc tacacgacgg 7560
ggagtcaggc aactatggat gaacgaaata gacagatcgc tgagataggt gcctcactga 7620
```

```
ttaagcattg gtaactgtca gaccaagttt actcatatat actttagatt gatttaaaac 7680
ttcattttta atttaaaagg atctaggtga agatcctttt tgataatctc atgaccaaaa 7740
tcccttaacg tgagttttcg ttccactgag cgtcagaccc cgtagaaaag atcaaaggat 7800
cttcttgaga tccttttttt ctgcgcgtaa tctgctgctt gcaaacaaaa aaaccaccgc 7860
taccagcggt ggtttgtttg ccggatcaag agctaccaac tctttttccg aaggtaactg 7920
gcttcagcag agcgcagata ccaaatactg tccttctagt gtagccgtag ttaggccacc 7980
acttcaagaa ctctgtagca ccgcctacat acctcgctct gctaatcctg ttaccagtgg 8040
ctgctgccag tggcgataag tcgtgtctta ccgggttgga ctcaagacga tagttaccgg 8100
ataaggcgca gcggtcgggc tgaacggggg gttcgtgcac acagcccagc ttggagcgaa 8160
cgacctacac cgaactgaga tacctacagc gtgagcattg agaaagcgcc acgcttcccg 8220
aagggagaaa ggcggacagg tatccggtaa gcggcagggt cggaacagga gagcgcacga 8280
gggagcttcc agggggaaac gcctggtatc tttatagtcc tgtcgggttt cgccacctct 8340
gacttgagcg tcgatttttg tgatgctcgt caggggggcg gagcctatgg aaaaacgcca 8400
gcaacgcggc ctttttacgg ttcctggcct tttgctggcc ttttgctcac atgttctttc 8460
ctgcgttatc ccctgattct gtggataacc gtattaccgc ctttgagtga gctgataccg 8520
ctcgccgcag ccgaacgacc gagcgcagcg agtcagtgag cgaggaagcg gaagagcgcc 8580
caatacgcaa accgcctctc cccgcgcgtt ggccgattca ttaatgcagg            8630
```

<210> 2
<211> 8630
<212> DNA
<213> Künstliche Sequenz

<220>
<223> Beschreibung der künstlichen Sequenz: Provirale Plasmid-DNA

<220>
<221> misc_feature
<222> (1)..(8630)
<223> Retroviraler Expressionsvektor SFbeta91mSAl

<220>
<221> misc_feature
<222> (1)..(160)
<223> Plasmid-Rückgrat (pUC)

<220>
<221> misc_feature
<222> (161)..(677)
<223> 5'-LTR

<220>
<221> 5'UTR
<222> (532)..(1219)

<220>
<221> mat_peptide
<222> (1220)..(5062)
<223> mSA1 mdr1 cDNA

<220>
<221> misc_feature
<222> (5215)..(5774)
<223> 3'-LTR

<220>
<221> misc_feature
<222> (5775)..(8630)
<223> Plasmid-Rückgrat (pUC)

<400> 2

```
tcgagggggg gcccggtcac gattagtcca atttgttaaa gacaggatat cagtggtcca 60
ggctctagtt ttgactcaac aatatcacca gctgaagcct atagagtacg agccatagat 120
agaataaaag attttattta gtctccagaa aaagggggga atgaaagacc ccacctgtag 180
gtttggcaag ctagcttaag taacgccatt ttgcaaggca tggaaaatac ataactgaga 240
atagagaagt tcagatcaag gttaggaaca gagagacagc agaatatggg ccaaacagga 300
tatctgtggt aagcagttcc tgccccgctc agggccaaga acagatggtc cccagatgcg 360
gtcccgccct cagcagtttc tagagaacca tcagatgttt ccagggtgcc ccaaggacct 420
gaaaatgacc ctgtgcctta tttgaactaa ccaatcagtt cgcttctcgc ttctgttcgc 480
gcgcttctgc tccccgagct caataaaaga gcccacaacc cctcactcgg cgcgccagtc 540
ctccgattga ctgagtcgcc cgggtacccg tattcccaat aaagcctctt gctgtttgca 600
tccgaatcgt ggactcgctg atccttggga gggtctcctc agattgattg actgcccacc 660
tcgggggtct ttcatttgga ggttccaccg agatttggag acccctgccc agggaccacc 720
gaccccccg ccgggaggta agctggccag cggtcgtttc gtgtctgtct ctgtctttgt 780
gcgtgtttgt gccggcatct agtgtttgcg cctgcgtctg tactagttgg ctaactagat 840
ctgtatctgg cggtcccgcg gaagaactga cgagttcgta ttcccggccg cagcccctgg 900
gagacgtccc agcggcctcg gggcccgtt ttgtggccca ttctgtatca gttaacctac 960
ccgagtcgga ctttttggag ctccgccact gtccgagggg tacgtggctt tgttgggga 1020
cgagagacag agacacttcc cgcccccgtc tgaatttttg ctttcggttt tacgccgaaa 1080
ccgcgccgcg cgtcttgtct gctgcagcat cgttctgtgt tgtctctgtc tgactgtgtt 1140
tctgtatttg tctgaaaatt agctcgacaa agttaactaa tagtccctct ctccaagctc 1200
acttacaggc gcggccgcca tggatcttga aggggaccgc aatggaggag caaagaagaa 1260
gaactttttt aaactgaaca ataaaagtga aaaagataag aaggaaaaga aaccaactgt 1320
cagtgtattt tcaatgtttc gctattcaaa ttggcttgac aagttgtata tggtggtggg 1380
aactttggct gccatcatcc atggggctgg acttcctctc atgatgctgg tgtttggaga 1440
aatgacagat atctttgcaa atgcaggaaa tttagaagat ctgatgtcaa acatcactaa 1500
tagaagtgat atcaatgata cagggttctt catgaatctg gaggaagaca tgaccaggta 1560
tgcctattat tacagtggaa ttggtgctgg ggtgctggtt gctgcttaca ttcaggtttc 1620
attttggtgc ctggcagctg gaagacaaat acacaaaatt agaaaacagt tttttcatgc 1680
tataatgcga caggagatag gctggtttga tgtgcacgat gttggggagc ttaacacccg 1740
acttacagat gatgtctcta agattaatga agttattggt gacaaaattg gaatgttctt 1800
tcagtcaatg gcaacatttt tcactgggtt tatagtagga tttacacgtg gttggaagct 1860
aacccttgtg attttggcca tcagtcctgt tcttggactg tcagctgctg tctgggcaaa 1920
gatactatct tcatttactg ataaagaact cttagcgtat gcaaaagctg gagcagtagc 1980
tgaagaggtc ttggcagcaa ttagaactgt gattgcattt ggaggacaaa agaaagaact 2040
tgaaaggtac aacaaaaatt tagaagaagc taaaagaatt gggataaaga aagctattac 2100
agccaatatt tctataggtg ctgctttcct gctgatctat gcatcttatg ctctggcctt 2160
ctggtatggg accaccttgg tcctctcagg ggaatattct attggacaag tactcactgt 2220
attcttttct gtattaattg gggcttttag tgttggacag gcatctccaa gcattgaagc 2280
atttgcaaat gcaagaggag cagcttatga aatcttcaag ataattgata ataagccaag 2340
tattgacagc tattcgaaga gtgggcacaa accagataat attaagggaa atttggaatt 2400
cagaaatgtt cacttcagtt acccatctcg aaaagaagtt aagatcttga agggcctgaa 2460
cctgaaggtg cagagtgggc agacggtggc cctggttgga aacagtggct gtgggaagag 2520
cacaacagtc cagctgatgc agaggctcta tgaccccaca gaggggatgg tcagtgttga 2580
tggacaggat attaggacca taaatgtaag gtttctacgg gaaatcattg gtgtggtgag 2640
tcaggaacct gtattgtttg ccaccacgat agctgaaaac attcgctatg gccgtgaaaa 2700
tgtcaccatg gatgagattg agaaagctgt caaggaagcc aatgcctatg actttatcat 2760
gaaactgcct cataaatttg acaccctggt tggagagaga ggggcccagt tgagtggtgg 2820
gcagaagcag aggatcgcca ttgcacgtgc cctggttcgc aaccccaaga tcctcctgct 2880
ggatgaggcc acgtcagcct tggacacaga aagcgaagca gtggttcagg tggctctgga 2940
taaggccaga aaaggtcgga ccaccattgt gatagctcat cgtttgtcta cagttcgtaa 3000
tgctgacgtc atcgctggtt tcgatgatgg agtcattgtg gagaaaggaa atcatgatga 3060
actcatgaaa gagaaaggca tttacttcaa acttgtcaca atgcagacag caggaaatga 3120
agttgaatta gaaaatgcag ctgatgaatc caaaagtgaa attgatgcct tggaaatgtc 3180
ttcaaatgat tcaagatcca gtctaataag aaaaagatca actcgtagga gtgtccgtgg 3240
atcacaagcc caagacagaa agcttagtac caaagaggct ctggatgaaa gtatacctcc 3300
agtttccttt tggaggatta tgaagctaaa tttaactgaa tggccttatt ttgttgttgg 3360
tgtattttgt gccattataa atggaggcct gcaaccagca tttgcaataa tattttcaaa 3420
gattataggg gtttttacaa gaattgatga tcctgaaaca aaacgacaga atagtaactt 3480
gttttcacta ttgtttctag cccttggaat tatttctttt attacatttt tccttcaagg 3540
tttcacattt ggcaaagctg gagagatcct caccaagcgg ctccgataca tggttttccg 3600
atccatgctc agacaggatg tgagttggtt tgatgacccct aaaaacacca ctggagcatt 3660
gactaccagg ctcgccaatg atgctgctca agttaaaggg gctataggtt ccaggcttgc 3720
tgtaattacc cagaatatag caaatcttgg gacaggaata attatatcct tcatctatgg 3780
```

ttggcaacta acactgttac tcttagcaat tgtacccatc attgcaatag caggagttgt 3840
tgaaatgaaa atgttgtctg gacaagcact gaaagataag aaagaactag aaggtgctgg 3900
gaagatcgct actgaagcaa tagaaaactt ccgaaccgtt gtttctttga ctcaggagca 3960
gaagtttgaa catatgtatg ctcagagttt gcaggtacca tacagaaact ctttgaggaa 4020
agcacacatc tttggaatta cattttcctt cacccaggca atgatgtatt tttcctatgc 4080
tggatgtttc cggtttggag cctacttggt ggcacataaa ctcatgagct ttgaggatgt 4140
tctgttagta ttttcagctg ttgtctttgg tgccatggcc gtggggcaag tcagttcatt 4200
tgctcctgac tatgccaaag ccaaaatatc agcagcccac atcatcatga tcattgaaaa 4260
aaccccttg attgacagct acagcacgga aggcctaatg ccgaacacat tggaaggaaa 4320
tgtcacattt ggtgaagttg tattcaacta tcccacccga ccggacatcc cagtgcttca 4380
gggactgagc ctggaggtga agaagggcca gacgctggct ctggtgggca gcagtggctg 4440
tgggaagagc acagtggtcc agctcctgga gcggttctac gaccccttgg cagggaaagt 4500
gctgcttgat ggcaaagaaa taaagcgact gaatgttcag tggctccgag cacacctggg 4560
catcgtgtcc caggagccca tcctgtttga ctgcagcatt gctgagaaca ttgcctatgg 4620
agacaacagc cgggtggtgt cacaggaaga gatcgtgagg gcagcaaagg aggccaacat 4680
acatgccttc atcgagtcac tgcctaataa atatagcact aaagtaggag acaaaggaac 4740
tcagctctct ggtggccaga aacaacgcat tgccatagct cgtgcccttg ttagacagcc 4800
tcatattttg cttttggatg aagccacgtc agctctggat acagaaagtg aaaaggttgt 4860
ccaagaagcc ctggacaaag ccagagaagg ccgcacctgc attgtgattg ctcaccgcct 4920
gtccaccatc cagaatgcag acttaatagt ggtgtttcag aatggcagag tcaaggagca 4980
tggcacgcat cagcagctgc tggcacagaa aggcatctat ttttcaatgg tcagtgtcca 5040
ggctggaaca aagcgccagt gaactctgac tgtatgagat gttaaatact ttttaatggg 5100
gatccaagct tatcgatagg cctaggccta tcgataggcc taggcctatc gataggccta 5160
acacgagcca tagatagaat aaaagatttt atttagtctc cagaaaaagg ggggaatgaa 5220
agaccccacc tgtaggtttg gcaagctagc tgcagtaacg ccattttgca aggcatggaa 5280
aaataccaaa ccaagaatag agaagttcag atcaagggcg ggtacatgaa aatagctaac 5340
gttgggccaa acaggatatc tgcggtgagc agtttcggcc ccggcccggg gccaagaaca 5400
gatggtcacc gcagtttcgg ccccggcccg aggccaagaa cagatggtcc ccagatatgg 5460
cccaaccctc agcagtttct taagacccat cagatgtttc caggctcccc caaggacctg 5520
aaatgaccct gcgccttatt tgaattaacc aatcagcctg cttctcgctt ctgttcgcgc 5580
gcttctgctt cccgagctct ataaaagagc tcacaacccc tcactcggcg cgccagtcct 5640
ccgattgact gagtcgcccg ggtacccgta ttcccaataa agcctcttgc tgtttgcatc 5700
cgaatcgtgg actcgctgat ccttgggagg gtctcctcag attgattgac tgcccacctc 5760
gggggtcttt cagtaggatc tcgaccgatg cccttgagag ccttcaaccc agtcagctcc 5820
ttccggtggg cgcggggcat gactatcgtc gccgcactta tgactgtctt ctttatcatg 5880
caactcgtag gacaggtgcc ggcagcgctc tgggtcattt tcggcgagga ccgctttcgc 5940
tggagcgcga cgatgatcgg cctgtcgctt gcggtattcg gaatcttgca cgccctcgct 6000
caagccttcg tcactggtcc cgccaccaaa cgtttcggcg agaagcaggc cattatcgcc 6060
ggcatggcgg ccgacgcgct gggctacgtc ttgctggcgt tcgcgacgcg aggctggatg 6120
gccttcccca ttatgattct tctcgcttcc ggcggcatcg ggatgcccgc gttgcaggcc 6180
atgctgtcca ggcaggtaga tgacgaccat cagggacagc ttcaaggatc gctcgcggct 6240
cttaccagcc taacttcgat cattggaccg ctgatcgtca cggcgattta tgccgcctcg 6300
gcgagcacat ggaacgggtt ggcatggatt gtaggcgcct gatgcggtat tttctcctta 6360
cgcatctgtg cggtatttca caccgcatat ggtgcactct cagtacaatc tgctctgatg 6420
ccgcatagtt aagccagccc cgacacccgc caacacccgc tgacgcgccc tgacgggctt 6480
gtctgctccc ggcatccgct tacagacaag ctgtgaccgt ctccgggagc tgcatgtgtc 6540
agaggttttc accgtcatca ccgaaacgcg cgagacgaaa gggcctcgtg atacgcctat 6600
ttttataggt taatgtcatg ataataatgg tttcttagac gtcaggtggc acttttcggg 6660
gaaatgtgcg cggaacccct atttgtttat ttttctaaat acattcaaat atgtatccgc 6720
tcatgagaca ataaccctga taaatgcttc aataatattg aaaaaggaag agtatgagta 6780
ttcaacattt ccgtgtcgcc cttattccct tttttgcggc attttgcctt cctgtttttg 6840
ctcacccaga aacgctggtg aaagtaaaag atgctgaaga tcagttgggt gcacgagtgg 6900
gttacatcga actggatctc aacagcggta agatccttga gagttttcgc cccgaagaac 6960
gttttccaat gatgagcact tttaaagttc tgctatgtgg cgcggtatta tcccgtattg 7020
acgccgggca agagcaactc ggtcgccgca tacactattc tcagaatgac ttggttgagt 7080
actcaccagt cacagaaaag catcttacgg atggcatgac agtaagagaa ttatgcagtg 7140
ctgccataac catgagtgat aacactgcgg ccaacttact tctgacaacg atcggaggac 7200
cgaaggagct aaccgctttt ttgcacaaca tgggggatca tgtaactcgc cttgatcgtt 7260
gggaaccgga gctgaatgaa gccataccaa acgacgagcg tgacaccacg atgcctgtag 7320
caatggcaac aacgttgcgc aaactattaa ctggcgaact acttactcta gcttcccggc 7380
aacaattaat agactggatg gaggcggata aagttgcagg accacttctg cgctcggccc 7440
ttccggctgg ctggtttatt gctgataaat ctggagccgg tgagcgtggg tctcgcggta 7500
tcattgcagc actggggcca gatggtaagc cctcccgtat cgtagttatc tacacgacgg 7560
ggagtcaggc aactatggat gaacgaaata gacagatcgc tgagataggt gcctcactga 7620

```
ttaagcattg gtaactgtca gaccaagttt actcatatat actttagatt gatttaaaac 7680
ttcattttta atttaaaagg atctaggtga agatcctttt tgataatctc atgaccaaaa 7740
tcccttaacg tgagttttcg ttccactgag cgtcagaccc cgtagaaaag atcaaaggat 7800
cttcttgaga tccttttttt ctgcgcgtaa tctgctgctt gcaaacaaaa aaaccaccgc 7860
taccagcggt ggtttgtttg ccggatcaag agctaccaac tctttttccg aaggtaactg 7920
gcttcagcag agcgcagata ccaaatactg tccttctagt gtagccgtag ttaggccacc 7980
acttcaagaa ctctgtagca ccgcctacat acctcgctct gctaatcctg ttaccagtgg 8040
ctgctgccag tggcgataag tcgtgtctta ccgggttgga ctcaagacga tagttaccgg 8100
ataaggcgca gcggtcgggc tgaacggggg gttcgtgcac acagcccagc ttggagcgaa 8160
cgacctacac cgaactgaga tacctacagc gtgagcattg agaaagcgcc acgcttcccg 8220
aagggagaaa ggcggacagg tatccggtaa gcggcagggt cggaacagga gagcgcacga 8280
gggagcttcc agggggaaac gcctggtatc tttatagtcc tgtcgggttt cgccacctct 8340
gacttgagcg tcgatttttg tgatgctcgt caggggggcg gagcctatgg aaaaacgcca 8400
gcaacgcggc ctttttacgg ttcctggcct tttgctggcc ttttgctcac atgttctttc 8460
ctgcgttatc ccctgattct gtggataacc gtattaccgc ctttgagtga gctgataccg 8520
ctcgccgcag ccgaacgacc gagcgcagcg agtcagtgag cgaggaagcg gaagagcgcc 8580
caatacgcaa accgcctctc cccgcgcgtt ggccgattca ttaatgcagg            8630
```

<210> 3
<211> 28
<212> DNA
<213> Künstliche Sequenz

<220>
<223> Beschreibung der künstlichen Sequenz: PCR Primer

<220>
<221> misc_feature
<222> (1)..(28)
<223> delta gag+

<400> 3

```
gtgccggcat ctaatgtttg cgcctgcg                                    28
```

<210> 4
<211> 46
<212> DNA
<213> Künstliche Sequenz

<220>
<223> Beschreibung der künstlichen Sequenz: PCR Primer

<220>
<221> misc_feature
<222> (1)..(46)
<223> delta gag/Not-

<400> 4

```
atagtttagc ggccgctaat tttcagacaa atacagaaac acagtc                46
```

<210> 5
<211> 42
<212> DNA
<213> Künstliche Sequenz

<220>
<223> Beschreibung der künstlichen Sequenz: PCR Primer

<220>
<221> misc_feature
<222> (1)..(42)
<223> delta gag/Xho-

<400> 5

gtccgctcga gctaattttc agacaaatac agaaacacag tc 42

<210> 6
<211> 47
<212> DNA
<213> Künstliche Sequenz

<220>
<223> Beschreibung der künstliche Sequenz: Annealing-Oligonukleotid

<220>
<221> misc_feature
<222> (1)..(47)
<223> SA-oligo(+)

<400> 6

tcgacaaagt taagtaatag tccctctctc caagctcact tacaggc 47

<210> 7
<211> 47
<212> DNA
<213> Künstliche Sequenz

<220>
<223> Beschreibung der künstlichen Sequenz: Annealing-Oligonukleotid

<220>
<221> misc_feature
<222> (1)..(47)
<223> SA-oligo(-)

<400> 7

ggccgcctgt aagtgagctt ggagagaggg actattactt aactttg 47

<210> 8
<211> 28
<212> DNA
<213> Künstlich Sequenz

<220>
<223> Beschreibung der künstlichen Sequenz: PCR Primer

<220>

<221> misc_feature
<222> (1)..(28)
<223> MUT/Leader-ATG/+

<400> 8

gtgccggcat ctagtgtttg cgcctgcg 28

<210> 9
<211> 28
<212> DNA
<213> Künstliche Sequenz

<220>
<223> Beschreibung der künstlichen Sequenz: PCR Primer

<220>
<221> misc_feature
<222> (1)..(28)
<223> MUT/Leader-ATG/-

<400> 9

cgcaggcgca aacactagat gccggcac 28

<210> 10
<211> 30
<212> DNA
<213> Künstliche Sequenz

<220>
<223> Beschreibung der künstlichen Sequenz: PCR Primer

<220>
<221> misc_feature
<222> (1)..(30)
<223> SD1+

<400> 10

gaagacatga ccagatatgc ctattattac 30

<210> 11
<211> 30
<212> DNA
<213> Künstliche Sequenz

<220>
<223> Beschreibung der künstlichen Sequenz: PCR Primer

<220>
<221> misc_feature
<222> (1)..(30)
<223> SD1-

<400> 11

gtaataatag gcatatctgg tcatgtcttc 30

<210> 12
<211> 29
<212> DNA
<213> Künstliche Sequenz

<220>
<223> Beschreibung der künstlichen Sequenz: PCR Primer

<220>
<221> misc_feature
<222> (1)..(29)
<223> SA1+

<400> 12

cattttccct tcaaggtttc acatttggc 29

<210> 13
<211> 29
<212> DNA
<213> Künstliche Sequenz

<220>
<223> Beschreibung der künstlichen Sequenz: PCR Primer

<220>
<221> misc_feature
<222> (1)..(29)
<223> SA1-

<400> 13

gccaaatgtg aaaccttgaa ggaaaaatg 29

<210> 14
<211> 30
<212> DNA
<213> Künstliche Sequenz

<220>
<223> Beschreibung der künstlichen Sequenz: PCR Primer

<220>
<221> misc_feature
<222> (1)..(30)
<223> AATAAA+

<400> 14

gatggcaaag aaattaagcg actgaatgtt 30

<210> 15
<211> 31
<212> DNA
<213> Künstliche Sequenz

<220>
<223> Beschreibung der künstlichen Sequenz: PCR Primer

<220>
<221> misc_feature
<222> (1)..(31)
<223> AATAAA-

<400> 15

gaacattcag tcgcttaatt tctttgccat c 31

<210> 16
<211> 36
<212> DNA
<213> Künstliche Sequenz

<220>
<223> Beschreibung der künstlichen Sequenz: PCR Primer

<220>
<221> misc_feature
<222> (1)..(36)
<223> eGFP+

<400> 16

gcggccgcca tggtgagcaa gggcgaggag ctgttc 36

<210> 17
<211> 34
<212> DNA
<213> Künstliche Sequenz

<220>
<223> Beschreibung der künstlichen Sequenz: PCR Primer

<220>
<221> misc_feature
<222> (1)..(34)
<223> eGFP-

<400> 17

ctcgagttat cgagatctga gtccggactt gtac 34

## Beschreibung

Gegenstand der Erfindung sind neuartige retrovirale Gentransfervektoren, vorzugsweise Expressionsvektoren, die sich aufgrund eines verminderten Anteiles an viralen Genen durch einen höheren Sicherheitsstandard und eine Expression nicht-viraler Nukleotidsequenzen in höheren Mengen auszeichen.

Die Expression fremder Proteine in pro- und eukaryontischen Zellen, d.h. von Proteinen, die in diesen Zellen normalerweise gar nicht oder nur in sehr geringen Mengen exprimiert werden, spielt eine wesentliche Rolle bei der Erforschung der Funktion von Proteinen, der Produktion von Proteinen sowie bei der Therapie von Krankheiten. Voraussetzung für die Expression eines fremden Genes ist es, daß die für das Protein kodierende Erbsubstanz in die Zielzelle eingeführt wird. Dies geschieht in der Regel mit Hilfe von sogenannten Vektoren. Vektoren sind DNA-Moleküle, in welche die für das zu exprimierende Protein kodierende DNA hineinkloniert wurde und die DNA Sequenzen enthalten, welche für die Expression des Proteins von Bedeutung sind.

Für den stabilen Gentransfer in Säugetierzellen sind retrovirale Vektoren auf der Grundlage von Maus-Leukämieviren (MLV) das gegenwärtig am häufigsten verwendete und am besten charakterisierte System (Miller, A.D. (1993) Methods Enzymol. 217: 581-599). Anwendungsgebiete retroviraler Vektoren sind beispielsweise die Überexpression von Proteinen mit dem Ziel der Gewinnung reiner Proteine, die Expressionsklonierung mit dem Ziel, neue Proteine zu identifizieren, sowie die stabile Expression eines Proteins in einer Körperzelle mit dem Ziel einer therapeutischen Wirkung des exprimierten Proteins. Maßgeblich für das therapeutische Potential des Gentransfers ist dabei das Niveau der Transgenexpression im bei der Krankheit relevanten Zellsystem. Für eine klinische Anwendung von Gentransfervektoren ist zudem die Sicherheit der Vektoren von entscheidender Bedeutung. Dabei muß zum einen die Expression viraler Genprodukte ausgeschlossen werden, da diese im Körper eine pathogene Wirkung entfalten können. Zum anderen soll eine Rekombination mit natürlich vorkommenden Viren ausgeschlossen werden, um zu verhindern, daß virale Proteine unter der Kontrolle der in den Vektor eingefügten regulatorischen Elemente exprimiert werden bzw. neuartige Viren mit unbekannten Eigenschaften entstehen.

Retrovirale Vektoren können in zwei Formen vorliegen, als provirale DNA oder als Vektor-RNA. Stabil im Genom der Zielzelle integriert ist die provirale Doppelstrang-DNA. Von dieser wird das sogenannte genomische Vektortranskript abgelesen, das wie eine zelluläre mRNA (Boten-RNA) aufgebaut ist, in virale Partikel verpackt wird und die Erbinformation des Retrovirus überträgt. Das genomische Vektortranskript wird nach retroviraler Infektion einer Zielzelle durch reverse Transkription in ein neues Provirus umgeschrieben und in das Genom der Zelle stabil integriert (Miller, A.D. (1993) Methods Enzymol. 217:581-599).

Das Provirus wird am 5'- und am 3'- Ende von "long terminal repeats" (LTR) flankiert, welche die Regionen U3, R und U5 umfassen (Abb. 1). Die U3-Region enthält Enhancer/Promotorsequenzen, die die Transkription des Vektors steuern. Die R-Region trägt das Polyadenylierungssignal. Die U5-Region enthält Sequenzen, die für die Integration des Retrovirus notwendig sind. Die kodierenden Sequenzen des exogenen Proteins liegen gewöhnlich zwischen 5'- und 3'-LTR im Vektor und werden von regulatorisch wichtigen Kontrollsequenzen flankiert, die für den Ablauf des retroviralen Lebenszyklus essentiell sind und zugleich die Halbwertszeit der RNA sowie die Effizienz der Translation des exogenen Proteins beeinflussen. Der Transkriptionsstart der RNA liegt an der Grenze zur R-Region des 5'-LTR. Das Transkriptionsende wird durch Polyadenylierungs- und Terminationssignale in der R-Region des 3'-LTR bestimmt. Am Ende der R-Region des 3'-LTR wird ein Polyadenosin-Schwanz angeheftet. Spleißsignale des Retrovirus/ Vektors können zu Transkripten mit internen Deletionen führen.

Der 5'-untranslatierte Bereich des retroviralen Vektors setzt sich zusammen aus (A.D. Miller (1993) a.a.O.):

- R-Region und U5-Region des 5'-LTR (ca. 150 Nukleotide, notwendig für reverse Transkription und Integration)
- Primerbindungsstelle (18 Nukleotide, notwendig für reverse Transkription)
- Leader-Region (bei gängigen retroviralen Vektoren mindestens 800 Nukleotide lang). Sie schließt sich an die Primerbindungsstelle an und reicht bis zum Start der kodierenden Sequenz. Die Leader-Region enthält das Verpackungs-und Dimerisierungssignal, welches für die Inkorporation der RNA in retrovirale Partikel notwendig ist. Am Beginn des Leaders liegt der retrovirale Spleißdonor, am Ende des Leaders kann ein kryptischer (d.h. nur in einem Teil der Transkripte erkannter) Spleißakzeptor liegen. Spleißdonor und Spleißakzeptor bestimmen Anfang und Ende der RNA-Sequenzen, die durch den Vorgang des Spleißens aus dem primären Transkript noch vor Export in das Zytoplasma entfernt werden können. Die Effizienz der Spleißreaktion hängt von der Eignung der Spleißsignale ab. Neben den Sequenzen von Spleißdonor und Spleißakzeptor bestimmen der vor dem Spleißakzeptor gelegene Polypyrimidintrakt und die anschließende sog. Verzweigungsstelle (Branch-site, s. unten) die Effizienz des Spleißens (Zhuang, Y.A. et al. (1989) Proc. Natl. Acad. Sci. USA, 86:2752-2756).

Die Effizienz der Translation wird durch die große Länge des 5'-untranslatierten Bereiches (5'-UTR) beeinträchtigt. Die Spleißsignale des Leaders können Anlaß zu verkürzten Transkripten mit deutlich kleineren 5'-UTR geben, die eine erhöhte Translationseffizienz aufweisen (Armentano, D. et al. (1987) J. Virol. 61:1647-1620; Bender, M.A. et al. (1987) J. Virol. 61:1639-1646; Krall, W.J. et al. (1996) Gene Ther. 3:37-48).

Der 3'-untranslatierte Bereich der RNA enthält den Polypurintrakt, welcher für die reverse Transkription notwendig ist, sowie die U3- und R-Region des 3'-LTR.

Nach Ablauf des retroviralen Lebenszyklus wird die U3-Region des 3'-UTR in beide LTRs kopiert. Sind in der U3-Region Enhancer/Promoterbereiche enthalten, so steuern diese die Transkription der Vektor-RNA in der infizierten Zelle (Baum, C. et al. (1995) J. Virol. 69:7541-7547). Das Ende der RNA ist der Polyadenosin-schwanz von ca. 200 Nukleotiden, der die Stabilität im Zytoplasma mitbestimmt.

Neben den Kontrollelementen enthalten Retroviren Nukleotidsequenzen, die für virale Proteine kodieren. Zu diesen zählen das *gag*-Gen, das für Strukturproteine des Virus kodiert, das *pro*-Gen, das für die Virion-Protease kodiert, das *pol*-Gen, das für die Reverse Transkriptase kodiert sowie das *env*-Gen, das für Virus-Hüllglykoproteine kodiert. Enthält der retrovirale Vektor eines dieser Gene nicht, so ist er replikationsinkompetent. Um infektiöse Viruspartikel aus einem repikationsinkompetenten retroviralen Vektor herzustellen, benötigt man einen Helfervirus und/oder eine verpackungskompetente Zellinie, welche die dem retroviralen Vektor fehlenden Eigenschaften zur Verfügung stellen.

Die in den retroviralen Vektoren enthaltenen viralen Sequenzen können Anlaß zur Rekombination mit komplementären Retroviren in der Verpackungszelle geben, wodurch replikationskompetente Retroviren entstehen können, die im Tierversuch Leukämien und Enzephalophatien auslösen können (Anderson, W.F. (1993) Hum. Gene Ther. 4: 311-321; Münk, C. (1997) PNAS 94: 5837-5842). Außerdem enthalten die residualen *gag*- und *pol*-Gene eine Vielzahl kryptischer Leserahmen, d.h. Nukleotidsequenzen, die in einem anderen Leserahmen als für das eigentliche Gen für eine Aminosäuresequenz kodieren, aber im Normalfall nicht abgelesen werden, da die Kontrollelemente und/oder das Startcodon fehlen. Dennoch ist nicht auszuschließen, daß von den offenen Leserahmen immunogene oder toxische Peptide in der Zielzelle erzeugt werden.

Die Zielzelle des retroviralen Expressionsvektors wird mit infektiösen Viruspartikeln transduziert. Diese Partikel werden mit Hilfe einer verpackungskompetenten Zellinie hergestellt. Dabei ist es wichtig, daß pro ml Zellkulturüberstand möglichst viele infektiöse Viruspartikel von den verpackungskompetenten Zellen produziert werden, daß also der Virustiter, angegeben in infektiösen Einheiten pro ml Kulturmedium, möglichst hoch ist.

Retrovirale Vektoren werden häufig auf der Grundlage von Kontrollelementen hergestellt, die Maus-Leukämieviren (MLV) entnommen werden (Miller, A.D., a.a.O.). Diese Vektoren werden auf MLV basierende Vektoren genannt. Ein zentrales Anwendungsgebiet dieser Gruppe von retroviralen Vektoren ist der Gentransfer in hämatopoetische Stammzellen (Dunbar, C.E. (1996) Ann. Rev. Med. 47: 11-20). Dabei sollen mit dem durch Transfer und Expression des vom Vektor kodierten Proteins therapeutische Wirkungen erzielt werden. Das Protein wird in den Zellen permanent produziert. Daher ist eine lang anhaltende Therapie denkbar, die direkt auf die der Krankheit zugrunde liegenden genetischen Defekte wirkt. Beispielsweise kann der Vektor Gene wie das menschliche *mdr1* (multidrug resistence 1) zum Schutz der Stammzellen gegen toxische Nebenwirkungen einer zytostatischen Pharmakotherapie (Stammzellschutz) tragen [Baum, C. et al. (1996) Gene Ther. 3: 1-3, Baum, C. et al. (1995) a.a.O.).

Im Stand der Technik sind retrovirale Vektoren beschrieben, bei denen das *gag*-Gen einschließlich einer unmittelbar 5' (d.h. stromaufwärts) des Startcodons des *gag*-Gens liegenden, 60 bp umfassenden Sequenz des 5'-UTR entfernt wurde. Mit diesen Vektoren wurden jedoch nur sehr niedrige Virustiter erhalten. Arbeiten von Armentano und Bender aus dem Jahre 1987 (Armentano, D. et al. (1987) J. Virol. 61: 1647-1650; Bender, M.A. et al. (1987) J. Virol. 61: 1639-1646) haben gezeigt, daß bei von MLV abgeleiteten retroviralen Vektoren der Virustiter höher ist, wenn ein Teil des *gag*-Genes (mindestens 400 bp) im Vektor belassen wird. Daraus wurde geschlossen, daß das für den retroviralen Titer maßgebliche Verpackungssignal nicht nur auf den 5' untranslatierten Bereich des Retrovirus begrenzt ist, sondern in die virale *gag*-Sequenz hineinreicht. Diese Meinung hat inzwischen auch in Lehrbüchern Niederschlag gefunden (Kriegler, M. (1990) Gene Transfer and Expression, Stockton Press, New York, S. 52). Aus diesem Grund werden im Stand der Technik keine retroviralen Vektoren hergestellt, die weniger als 400 bp des *gag*-Genes enthalten oder bei denen die *gag*-Sequenzen ganz entfernt wurden. Die dem Stand der Technik entsprechenden Vektoren werden auch als (gag+)-Vektoren bezeichnet.

In Retroviren hängt das Translationsniveau retroviraler Sequenzen von der Anwesenheit passender Spleißsignale und der Lage des Startkodons in der gespleißten RNA ab. Unter einem Spleißsignal versteht man eine Nukleotidsequenz, die das Spleißen der bei der Transkription gebildeten RNA reguliert. Diese Spleißsignale bestehen je nach Virusstamm, der dem Vektor zugrunde liegt, aus unterschiedlichen Nukleotidsequenzen. Ein von einem bestimmten Virus abstammendes Spleißsignal ist aber in der Regel auch in Vektoren aktiv, die von anderen Viren abstammen (Bowtell, D.D. et al. (1988) J. Virol. 62: 2464-2473). Alle Spleißsignale haben eine Konsensussequenz gemeinsam, die eine "branch site" enthält. Als "branch site" wird das Nukleophil bezeichnet, das für den ersten Transesterifizierungsschritt der Spleißreaktion notwendig ist. Als "branch site" fungiert gewöhnlich ein Adenosin-Nukleotid. In von MLV abgeleiteten Viren hängt das Translationsniveau retroviraler *env*-Sequenzen von der Anwesenheit passender Spleißsignale und der Lage des Startkodons in der gespleißten RNA ab. Diese Beobachtung lag der Entwicklung der MFG-Vektoren zugrunde (Riviere, I. et al. (1995) PNAS 92: 6733-6737). Die Abkürzung MFG ist von einem Eigennamen abgeleitet, der in der Literatur nicht erläutert ist (Dranoff, G. et al. (1993) Proc. Natl. Acad. Sci. USA 90:

3539-3543). MFG-Vektoren sind als (*gag*+)-Vektoren aufgebaut und enthalten zusätzlich noch ein Fragment des retroviralen *pol*-Gens, das den Spleißakzeptor des Moloney MLV trägt. Die kodierende Sequenz des zu übertragenden Gens wird exakt an die Stelle und als Ersatz des retroviralen *env*-Gens plaziert (Abb. 1). Im Vergleich zu anderen MLV-Vektoren mit identischen Enhancer-Promotorsequenzen zeigen MFG-Vektoren eine erhöhte Expression des exogenen Gens (Krall, W.J. et al. (1996) Gene Ther. 3: 37-48). Jedoch werden in MFG-Vektoren nicht mehr als 50 % der hergestellten Transkripte gespleißt, so daß nach wie vor ein großer Anteil der Transkripte für die Translation verlorengeht. Auch hinsichtlich des Sicherheitsaspekts weisen MFG-Vektoren Probleme auf, da sie große Anteile der viralen *gag*- und *pol*-Gene tragen, um eine hohe Verpackungseffizienz und gute Spleißraten zu erzielen. Diese viralen Sequenzen können Anlaß zur Rekombination mit komplementären Retroviren in der Verpackungszelle geben (s.o.). MFG-Vektoren sind daher für den experimentellen Einsatz nur bedingt und für eine therapeutische Anwendung wenig geeignet, da sie aufgrund des immunogenen bzw. toxischen Potentials zu große Sicherheitsrisiken in sich bergen.

Eine weitere Gruppe von auf MLV basierenden retroviralen Vektoren sind die LX-Vektoren. Auch LX-Vektoren sind (*gag*+)-Vektoren. LX steht für LTR-Gen x (Miller, A.D. and G.J. Rosman (1987) Biotechniques 7:980-990). Bei LX-Vektoren wird das Transgen mitten in die *gag*-Region eingebaut (ca. 400 Basenpaare 3' des *gag*-Startkodons, das zu einem Stopkodon mutiert wurde). Die übrigen *gag*-, *pol*- und *env*-Sequenzen sind komplett entfernt worden. Die LX-Vektoren tragen damit weniger virale Sequenzen als die MFG-Vektoren. Allerdings ist die Effizienz des Spleißens - und damit auch der Translation des Transgens - bei den LX-Vektoren in der Regel geringer als bei den MFG-Vektoren (Armentano, D. et al. (1987) J. Virol. 61:1647-1620; Bender, M.A. et al. (1987) J. Virol. 61:1639-1646; Krall, W.J. et al. (1996) Gene Ther. 3:37-48).

Im Stand der Technik haben sich bislang LX- oder MFG- Vektoren durchgesetzt, die außer den für das exogene Protein kodierenden Nukleotidsequenzen auch Sequenzen für residuale virale Genprodukte sowie mindestens 400 bp des *gag*-Genes enthalten. Wie bereits erwähnt ergibt sich daraus der Nachteil, daß bei diesen Vektoren stets die Gefahr besteht, daß auch virale Proteine oder Peptide exprimiert werden und eine Rekombination mit anderen Viren erfolgt. Außerdem verringern die mindestens 400 bp des *gag*-Genes die Klonierungskapazität, d.h. die maximale Länge der exogenen Nukleotidsequenz des Vektors, da ein retroviraler Vektor maximal 10 kb umfassen kann. Ein weiterer Nachteil der MFG oder LX Vektoren liegt darin, daß mindestens die Hälfte der Transkripte nicht gespleißt wird und somit nicht für die Translation zur Verfügung steht.

Aufgabe der vorliegenden Erfindung ist es daher, retrovirale Vektoren zur Verfügung zu stellen, die die genannten Nachteile der im Stand der Technik verwendeten Vektoren nicht aufweisen. Insbesondere ist es Aufgabe der vorliegenden Erfindung, Vektoren bereitzustellen, die gegenüber den bislang bekannten Vektoren einen höheren Sicherheitsstandard erfüllen. Insbesondere soll die Gefahr minimiert werden, daß virale Proteine in den Zielzellen erzeugt werden; vorzugsweise sollen keine immunogenen oder toxischen Proteine exprimiert werden. Wesentliches Ziel ist es, die Wahrscheinlichkeit für eine Rekombination mit anderen Viren zu vermindern bzw. ganz auszuschließen. Ferner ist es Aufgabe der Erfindung, retrovirale Expressionsvektoren bereitzustellen, die gegenüber den im Stand der Technik verwendeten Vektoren einen vergleichbaren Virustiter und eine höhere Klonierungskapazität aufweisen und die Expression nicht-viraler Nukleotidsequenzen in hohen Mengen gestatten.

Die Aufgabe wird erfindungsgemäß dadurch gelöst, daß erstmals retrovirale Vektoren zur Verfügung gestellt werden, die weniger als 400 bp der für Gag kodierenden Nukleotidsequenz enthalten. Vorzugsweise enthalten die erfindungsgemäßen Vektoren weniger als 200 bp oder weniger als 80 bp einer für gag kodierenden Nukleotidsequenz. Gemäß einer bevorzugten Ausführungsform der vorliegenden Erfindung enthalten die retroviralen Vektoren keine für Gag kodierenden Sequenzen oder Sequenzabschnitte. Besonders bevorzugt sind Vektoren, bei denen die für virale Proteine kodierenden Sequenzen vollständig entfernt sind. Vorzugsweise wird der retrovirale Vektor als Expressionsvektor verwendet.

Gegenstand der Erfindung ist somit ein retroviraler Vektor, der eine Nukleotidsequenz N der allgemeinen Formel

5'-Ende -- [5'-UTR] - [gag] - [ex] - [vir] -- 3'-Ende

enthält, wobei
[5'-UTR] eine nicht-kodierende Nukleotidsequenz darstellt, die in Retroviren unmittelbar 5', d.h., stromaufwärts des Startcodons des *gag*-Genes liegt,
[gag] einen Abschnitt der Nukleotidsequenz darstellt, die für das *gag*-Gen kodiert,
[ex] eine nicht-virale Nukleotidsequenz darstellt und
[vir] eine retrovirale Nukleotidsequenz darstellt,
und der dadurch gekennzeichnet ist,
daß die Anzahl an Nukleotiden in [gag] weniger als 400 bp umfaßt.

Im Rahmen der vorliegenden Erfindung hat sich überraschenderweise gezeigt, daß im Gegensatz zur herrschenden Meinung mit dem erfindungsgemäßen Konstrukt, das weniger als 400 bp des *gag*-Gens und gemäß einer besonders bevorzugten Ausführungsform keine für das *gag*-Gen kodierenden Nukleotide umfaßt, hohe Virustiter erzielt werden können und eine hohe Expression der nicht-viralen Nukleotidsequenzen möglich ist. Durch die Reduktion des *gag*-Anteiles ist die Klonierungskapazität und die Sicherheit der erfindungsgemäßen Vektoren erhöht.

Als [5'-UTR] kommen alle Nukleotidsequenzen in Betracht, die in Retroviren oder in von Retroviren abgeleiteten retroviralen Konstrukten unmittelbar 5', d.h. stromaufwärts des Startcodons des gag-Genes liegen, wobei in den retroviralen Konstrukten diese Nukleotidsequenzen nicht derart verändert sein dürfen, daß die Veränderung im Vergleich zu der nicht veränderten Sequenz zu einer deutlichen (mehr als eine Größenordnung) Reduktion des Virustiters oder der Translationseffizienz führt. Erfindungsgemäß eingeschlossen sind Mutationen und kleinere Deletionen, die nicht zu einer Verringerung des Virustiters oder der Translationseffizienz führen. Erfindungsgemäß ausgeschlossen ist eine Deletion eines mindestens 60 bp umfassenden Sequenzabschnittes, der unmittelbar vor dem Startcodon des gag-Genes liegt. Erfindungsgemäß bedeutet "unmittelbar vor dem Startcodon", daß sich zwischen diesem Sequenzabschnitt und dem Startcodon des *gag*-Genes außer eingebauten Spleißakzeptornukleotiden und/oder Polylinkerfragmenten keine weiteren Nukleotide befinden.

Gemäß einer besonderen Ausführungsform der Erfindung enthält der [5'-UTR] mindestens einen Sequenzabschnitt, der einen Spleißakzeptor darstellt. Der Spleißakzeptor kann aus dem FMEV-Vektor SF1MSN, aber auch von anderen retroviralen oder nicht-retroviralen Vektoren, Viren wie beispielsweise HIV oder auch aus intronhaltigen Eukaryontengenen stammen. Der Spleißakzeptor kann dabei ein großes genomisches Restriktionsfragment des viralen *pol*-Genes sein. Besonders bevorzugt wird ein synthetisch erzeugtes Oligonukleotid, das nur den minimalen Konsens für den Spleißakzeptor (inklusive der sogenannten Branch-Site) repräsentiert und eine Länge von 10-100, vorzugsweise von 10-50, besonders bevorzugt von 20-50 Basenpaaren hat.

In einer weiteren bevorzugten Ausführungsform enthält [5'-UTR] an keiner Stelle das Nukleotidtriplett AUG (Adenin-Uracil-Guanin), das in Retroviren als Startcodon dient. Dadurch wird ausgeschlossen, daß kryptische offene Leserahmen im [5'-UTR] translatiert werden. Dies führt ebenfalls zu einer Erhöhung des Sicherheitsstandards. Die AUG-deletierten Nukleotidsequenzen sind beispielsweise durch eine Mutation mittels "site directed mutagenesis" erhältlich (s. Beispiel 4).

Erfindungsgemäß kann 5'-UTR eine Leadersequenz enthalten. Diese kann gemäß einer besonderen Ausführungsform aus dem MESV-Virus stammen (Grez, M. et al., Proc. Natl. Acad. Sci. USA 87:9202-9206 (1990)) es ist aber ebenso denkbar, die Leadersequenz von MoMoLV oder MoMuSV zu verwenden (Miller, A.D. and Rosmann, G. J., a.a.O.). Die Viren, deren 5'-Leader im erfindungsgemäßen retroviralen Vektor verwendet werden können, schließen ferner beispielsweise "spleen necrosis virus" (Olson P. et al. (1994) J. Virol. 68: 7060-7066) oder "Harvey murine sarcoma virus" (Berlioz, C. et al. (1995) J. Virol. 69: 6400-6407) ein, sind aber nicht auf diese beschränkt.

Das Sequenzelement [gag] kann beliebigen Abschnitten der für das Gag-Protein kodierenden Nukleotidsequenz entnommen werden. Erfindungsgemäß enthält [gag] weniger als 400 bp. Bevorzugt enthält [gag] weniger als 200 bp, vorzugsweise weniger als 80 bp.

Gemäß einer besonders bevorzugten Ausführungsform der Erfindung enthält der retrovirale Expressionsvektor 5' von [ex] keine Nukleotide oder Sequenzfragmente, die aus dem *gag*-Gen stammen. Dies bedeutet, daß die nicht-virale Nukleotidsequenz [ex] unmittelbar 3' von [5'-UTR] liegt. Erfindungsgemäß bedeutet "unmittelbar 3' von dem [5'-UTR]", daß sich zwischen diesem Sequenzabschnitt und dem ersten Nukleotid von [ex] außer eingebauten Spleißakzeptornukleotiden und/oder Polylinkerfragmenten keine weiteren Nukleotide befinden. Die nicht-virale Nukleotidsequenz [ex] ist dann an die Stelle des *gag*-Genes in den retroviralen Expressionsvektor eingebaut. Dadurch wird die Architektur eines nativen Retrovirus nachvollzogen. Diese genaue Nachahmung der Struktur des nativen Retrovirus führt zu einer erhöhten Expression des Gens, die durch Einführen eines künstlichen Spleißakzeptors noch gesteigert werden kann.

Diese besonders bevorzugte Ausführungsform, bei der [ex] das *gag*-Gen ersetzt, ist erhältlich, indem die residualen *gag*-Anteile mittels PCR entfernt werden (siehe Beispiel 1). Diese Techniken sind dem Fachmann bekannt (Ausubel, I. et al. (1994) Current protocols in Molecular Biology. John Wiley & Sons, New York). Dies führt zu einem Vektor, der anstelle des *gag*-Genes eine "multiple cloning site" trägt. Eine multiple cloning site ist eine Nukleotidsequenz, die verschiedene Schnittstellen für Restriktionsenzyme enthält. Mittels dem Fachmann bekannter Techniken kann dann die gewünschte nicht-virale Nukleotidsequenz [ex] unmittelbar an das 3'-Ende des [5'-Bereichs] kloniert werden.

Erfindungsgemäß befindet sich die Nukleotidsequenz [ex] zwischen [gag] und [vir] und stellt eine nicht-virale Nukleotidsequenz dar. Bevorzugt enthält sie mindestens eine Nukleotidsequenz, die für ein nicht-virales Protein kodiert. Die Sequenz [ex] kann auch eine nicht-virale RNA-Sequenz umfassen. Gemäß einer Ausführungsform der Erfindung enhält die nicht-virale Nukleotidsequenz [ex] eine Nukleotidsequenz, die für das MDR1-Gen kodiert. Gemäß einer weiteren Ausführungsform enthält [ex] eine Nukleotidsequenz, die für das als zytoplasmatischen Marker geeignete Enhanced Humanized Green Fluorescent Protein (EGFP; Cormack, B.P. et al. (1996) Gene 173:33-38) kodiert.

Andere erfindungsgemäß geeignete Sequenzen schließen ein, sind aber nicht auf die genannten Beispiele beschränkt:

- Gene, die wie EGFP als Zellmarker verwendet werden (z.B. Oberflächenproteine wie human low affinity nerve growth factor receptor (Fehse, B. et al. (1997) Hum. Gene Ther. 8: 1815-1824))
- Gene, die wie MDR1 Resistenz gegen zytotoxische Pharmaka vermitteln können (z.B. Dehydrofolatreduktase; Zhao, S.C. et al. (1997) Hum. Gene Ther. 8: 903-909)
- Gene, die angeborene Stoffwechselerkrankungen korrigieren können (z.B. a-L-Iduronidase für die Therapie des Hurler-Scheie-Syndroms; Huang, M.M. et al. (1997) Gene Ther. 4: 1150-1159).

Da die Art des Gens keinen Einfluß auf die erfindungsgemäße Verbesserung der Vektoren hat, sind potentiell alle Sequenzen als [ex] interessant, die in retroviralen Vektoren übertragen werden. In der bevorzugten Ausführungsform der Erfindung wird durch die Elimination aller Virus-kodierenden Sequenzen die Klonierungskapazität für die zu übertragenden Gene um ca. 400 Basenpaare gesteigert.

Die für nicht-virale Proteine kodierenden Nukleotidsequenzen können entweder in Leserichtung, d.h. in 5' → 3'- Richtung oder in entgegengesetzter Orientierung, d.h. in 3' → 5'- Richtung in die nicht-virale Nukleotidsequenz [ex] kloniert sein. Gemäß einer besonderen Ausführungsform stellt das erste Nukleotid des Startcodons der für das nicht-virale Protein kodierenden Nukleotidsequenz das 5'-Ende von [ex] dar. Die nicht-virale Nukleotidsequenz [ex] kann außer dem für Proteine kodierenden Bereich ferner regulatorische Nukleotidsequenzen umfassen. Enthält die Nukleotidsequenz [ex] mehrere Nukleotidsequenzen, die für nicht-virale Proteine kodieren, so können diese Nukleotidsequenzen entweder direkt aufeinander folgen oder durch regulatorische Nukleotidsequenzen voneinander getrennt sein. Unter regulatorischen Nukleotidsequenzen sind dabei erfindungsgemäß alle Nukleotidsequenzen zu verstehen, die die Expression der nicht-viralen Proteine beeinflussen können. Dazu zählen insbesondere Spleißakzeptorstellen. Weitere Möglichkeiten für die Verknüpfung von Transkriptionseinheiten in [ex] sind interne Ribosomeneintrittsignale, interne Promotoren oder Fusionsgene (Hildinger, M. et al (1998) Hum. Gene Ther. 9: 33-42).

Wichtige regulatorische Nukleotidsequenzen sind ferner Promotor- und Enhancersequenzen. Die Promotor- und Enhancersequenzen können aus beliebigen viralen oder nicht-viralen Genen stammen. Die Promotoren stammen vorzugsweise aus MLV (Baum, C. et al. (1995) J. Virol. 69:7541-7547) sind jedoch nicht darauf beschränkt. Besonders geeignet sind Promotor- und Enhancersequenzen, die für die jeweilige Zielzelle spezifisch sind, weil so eine zellspezifische Expression möglich ist.

Die Promotor- und Enhancersequenzen können sowohl am 5'-Ende als auch am 3'-Ende von [ex] liegen. Ebenso können sie an jeder Position in [ex] liegen, auch innerhalb der für nicht-virale Proteine kodierenden Nukleotidsequenzen.

Gemäß einer weiteren Ausführungsform der Erfindung weisen die Nukleotidsequenzen, die für nicht-virale Proteine kodieren, solche Mutationen auf, die, ohne Veränderung der durch die nicht-virale Nukleotidsequenz kodierten Proteinsequenz, zur Entfernung kryptischer Spleißstellen und/oder kryptischer poly-A-Stellen führen. Dadurch wird vermieden, daß die durch Transkription der für die Proteine codierenden nicht-viralen Nukleotidsequenzen entstehenden mRNAs falsch gespleißt werden oder zu kurz sind, wenn der poly-A-Schwanz zu früh angehängt wird (McIvor, R. S. (1990) Virology 176:652-655; Johnson, J.J. et al. (1995) Hum. Gene Ther. 6: 611-623). Dabei ist site directed mutagenesis für das Einführen von Mutationen die aktuell gängige und vom Fachmann bevorzugte Technik. Erfindungsgemäß können aber auch andere Techniken verwendet werden. Andere Mutationen, die erfindungsgemäß interessant sind, beinhalten die Entfernung von ATG-Triplets, die zu einem fehlerhaften Start der Translation der Sequenz in [ex] führen können, die Verbesserung des Translationsstartes der Sequenz in [ex] durch Optimierung der sog. Kozak-Konsensussequenz (Krall, W.J. et al. (1996) Gene Ther. 3:37-48), oder die Einführung von Signalen, die für einen verbesserten Kernexport der RNA führen (Pasquinelli, A.E. et al. (1997) EMBO J. 16: 7500-7510); letztere können auch an anderer Stelle als in [ex] in den Vektor eingebracht werden.

Erfindungsgemäß stellt [vir] eine retrovirale Sequenz dar, die für ein oder mehrere virale Proteine oder für Teile davon kodierende Nukleotidsequenzen enthalten kann. Im Rahmen der vorliegenden Erfindung sind die für virale Proteine kodierenden Sequenzabschnitte im retroviralen Vektor vorzugsweise in Anzahl und Länge vermindert. Insbesondere enthält der Vektor vorzugsweise keine Sequenzen oder Sequenzabschnitte, die für das Pol-Protein, das Pro-Protein, das Env-Protein und/oder andere virale Proteine codieren. Erfindungsgemäß bevorzugt ist ein Expressionsvektor, der keine für das Gag-Protein, besonders bevorzugt keine für das Gag-Protein oder für andere virale Proteine kodierende Nukleotidsequenzen enthält. Das heißt, der retrovirale Vektor enthält insgesamt keine Nukleotidsequenzen, die für residuale virale Proteine kodieren. Ganz besonders bevorzugt ist ein retroviraler Expressionsvektor, der sowohl keine für residuale virale Proteine oder für Teile davon kodierenden Sequenzabschnitte und, außer in [ex], an keiner Stelle das Nukleotidtriplett AUG enthält. Dadurch wird sichergestellt, daß sowohl keine residualen viralen Proteine oder Teile davon als auch keine kryptischen Peptide produziert werden. Der Ausschluß von Nukleotidsequenzen, die für virale Proteine kodieren, stellt sicher, daß das auf Sequenzhomologie beruhende Rekombinationsrisiko mit MLV-Sequenzen in der Verpackungszelle minimiert wird. Ebenso wird die Gefahr einer aberranten Translation viraler Peptide in der Zielzelle reduziert. Weiterhin wird der Sicherheitstandard dadurch erhöht, daß der retrovirale Vektor kein Startcodon mit der Sequenz AUG enthält.

Stromabwärts der Nukleotidsequenz N, d.h. 3', liegt bei den erfindungsgemäßen Vektoren im allgemeinen ein 3'-LTR. Gemäß einer besonderen Ausführungsform kann das 3'-LTR aus spleen focus forming virus (SFFVp) verwendet werden. Die Viren, deren 3'-LTR verwendet werden kann, schließen ferner andere MLV wie Friend-MLV, MPSV oder MoMLV (Baum, C. et al. (1995) J. Virol. 69:7541-7547), sind aber nicht auf diese beschränkt.

Gemäß einer besonders bevorzugten Ausführungsform sind die erfindungsgemäßen retroviralen Vektoren die bei der Deutschen Sammlung von Mikroorganismen und Zellkulturen, Mascheroder Weg 1b, 38124 Braunschweig hinterlegten Vektoren SFβ71m4 (DSM 12066) und SFβ91mSA1 (DSM 12065) oder davon abgeleitete Vektoren.

Der unter DSM 12065 hinterlegte Vektor zeichnet sich dadurch aus, daß sämtliche Nukleotidsequenzen entfernt wurden, die für virale Proteine kodieren. Am 5'-Ende der proviralen DNA befinden sich die U3-, R- und U5-Regionen des 5'-LTR. Im 5'-LTR liegt die CAP-Stelle, an der die Transkription der viralen mRNA beginnt. 3' vom 5'-LTR liegt eine Nukleotidsequenz, die einen Spleißakzeptor (SA), eine Verpackungsregion (ψ) und einen Spleißdonor (SD) enthält. Das Nukleotid 271 (gezählt ab CAP-Stelle) wurde mutiert, um ein kryptisches AUG zu entfernen. An der Stelle, an der sich die für das *gag*-Gen kodierenden Sequenzen befanden, befindet sich die Variante mSA1 des MDR1 Gens, die sich dadurch auszeichnet, daß an Position 2320 (gezählt ab Startcodon des MDR1-Gens) ein kryptischer Spleißakzeptor mutiert wurde. 3' der MDR1-Variante mSA1 befindet sich der 3'-LTR mit den U3-, R- und U5-Regionen (vgl. Fig. 2).

Der unter DSM 12066 hinterlegte Vektor zeichnet sich dadurch aus, daß sämtliche Nukleotidsequenzen entfernt wurden, die für virale Proteine kodieren. Am 5'-Ende der proviralen DNA befinden sich die U3-, R- und U5-Regionen des 5'-LTR. Im 5'-LTR liegt die CAP-Stelle, an der die Transkription der viralen RNA beginnt. 3' vom 5'-LTR liegt eine Nukleotidsequenz, die einen Spleißakzeptor (SA), eine Verpackungsregion (ψ) und einen Spleißdonor (SD) enthält. An der Stelle, an der sich die für das *gag*-Gen kodierenden Sequenzen befanden, befindet sich die Variante m4 des MDR1 Gens, die sich dadurch auszeichnet, daß an Position 339 (gezählt ab Startcodon des MDR1-Gens) ein kryptischer Spleißdonor, an Position 2320 ein kryptischer Spleißakzeptor und an Position 3303 ein kryptisches Poly (A)-Signal mutiert wurde. 3' der MDR1-Variante m4 befindet sich der 3'-LTR mit den U3-, R- und U5-Regionen (vgl. Fig. 2).

Erfindungsgemäß ist ferner ein Vektor bevorzugt, der von dem unter DSM 12066 hinterlegten Vektor abgeleitet ist und bei dem zusätzlich an Position 271 (gezählt ab CAP-Stelle) ein kryptisches AUG mutiert wurde.

Die drei letztgenannten Vektoren, d.h. die unter DSM 12065 und 12066 hinterlegten Vektoren sowie der von DSM 12066 abgeleitete Vektor, eignen sich in besonders bevorzugter Weise zur Transfektion hämatopoietischer Stammzellen im Rahmen einer Gentherapie, um eine Resistenz der hämatopoietischen Stammzellen bei einer Chemotherapie zu erzielen (s. unten).

Gegenstand der vorliegenden Erfindung ist ferner ein Verfahren zur Herstellung eines infektiösen Viruspartikels. Die dem Verfahren zugrundeliegenden Techniken sind dem Fachmann bekannt (Ausubel, I. et al. (1994) Current protocols in Molecular Biology. John Wiley & Sons, New York). Mit Hilfe einer verpackungskompetenten Zellinie und/oder eines Helfervirus werden infektiöse Viruspartikel hergestellt, die den erfindungsgemäßen retroviralen Vektor enthalten. Das Helfervirus wird als replikationsinkompetentes Partikel von den Verpackungszellen produziert. Das Genom des Helfervirus ist gentechnisch dafür so verändert worden, daß die Virusprotein-kodierende RNA des Helfervirus nicht in die Viruspartikel verpackt werden kann (Miller, A.D. et al. (1993) Methods Enzymol. 217: 581-599).

Gegenstand der vorliegenden Erfindung ist ferner eine Wirtszelle, die mit dem erfindungsgemäßen retroviralen Vektor transfiziert ist. Bevorzugt wird die Wirtszelle mit einem infektiösen Viruspartikel infiziert (s.o.), das den erfindungsgemäßen retroviralen Vektor enthält. Diese Wirtszelle kann ausgewählt werden aus verfügbaren immortalisierten hämatopoetischen Zellinien (Baum, C et al. (1995) J. Virol. 69:7541-7547) oder primären menschlichen Blutzellen (Eckert, H.G. et al. (1996) Blood 88:3407-3415), ist aber nicht auf diese beschränkt. Vorzugsweise ist die Wirtszelle eine K-562 Zelle (menschliche Erythroleukämiezelle; Lozzio, C.B. et al. (1976) Cancer Res. 36:4657-4662), die mit dem Vektor SFβ71m4 (DSM Hinterlegungsnummer DSM 12066) oder mit dem Vektor SFβ91mSA1 (DSM Hinterlegungsnummer DSM 12065) transfiziert ist.

Weiterhin ist erfindungsgemäß eingeschlossen ein Verfahren zur Herstellung von Proteinen, bei dem man eine Wirtszelle, die mit einem erfindungsgemäßen retroviralen Vektor oder einem erfindungsgemäßen Viruspartikel transfiziert worden ist, in einem geeigneten Medium unter Bedingungen kultiviert, die zur Expression der retroviralen Proteine notwendig sind, für die die Nukleotidsequenzen in der Nukleotidsequenz [ex] kodieren. Diese Bedingungen können das Kultivieren in Zellulturstandardmedien wie Dulbecco's modified essential medium (DMEM) umfassen, die mit tierischem Serum (beispielsweise foetalem Kälberserum) supplementiert sind (Ausubel et al., a.a.O.). Danach wird das erzeugte Protein mit Hilfe von Techniken gereinigt, die dem Fachmann bekannt sind (Ausubel et al., a.a.O.), indem man es von den Zellen und dem Medium abtrennt.

Erfindungsgemäß kann der retrovirale Vektor therapeutisch angewendet werden. Dies bedeutet, daß er Bestandteil eines pharmazeutischen Präparats ist, welches zusätzlich pharmazeutisch verträgliche Hilfs- und/oder Trägerstoffe enthält.

Gemäß einer besonderen Ausführungsform der Erfindung findet der retrovirale Gentransfer- bzw. Expressionsvektor Verwendung in der Gentherapie. Dies schließt ein, daß der Vektor in einem pharmazeutischen Präparat derart vorliegt, daß er in die Zielzelle eingeführt werden kann. Bevorzugt liegt der erfindungsgemäße retrovirale Vektor in einem infektiöses Viruspartikel verpackt vor. Bevorzugt wird der retrovirale Vektor zur Transfektion hämatopoetischer Stammzellen verwendet (Baum, C. et al. (1997) in: Concepts in Gene Therapy (M. Strauss, W. Barranger, eds.), De Gruyter, Berlin, S. 233-266).

Ferner findet der erfindungsgemäße retrovirale Vektor Verwendung zur Expressionsklonierung von Genen. Dazu wird eine cDNA Bibliothek, in der das gesuchte Gen enthalten ist, in den erfindungsgemäßen retroviralen Epressionsvektor kloniert. Nach Verpackung des Vektors in infektiöse Partikel und Infizierung von Wirtszellen mit den Partikeln kann die Wirtszelle, die das gesuchte Protein exprimiert und damit das gesuchte Gen enthält, beispielsweise mittels Antikörpern oder über funktionelle Tests nachgewiesen werden.

Erfindungsgemäß kann der retrovirale Vektor ferner zur Expression und/oder Überexpression von Proteinen oder von RNA verwendet werden. Zu diesem Zweck wird das zu exprimierende Gen oder die RNA in den erfindungsgemäßen retroviralen Vektor kloniert. Nach Verpackung in infektiöse Viruspartikel und Infektion einer geeigneten Wirtszelle mit den Partikel kann das gewünschte Protein nach bekannten Verfahren produziert werden.

Die Erfindung wird nachfolgend anhand von Beispielen, Figuren und Sequenzprotokollen erläutert.

**Beispiel 1**

Klonierung eines gag-deletierten retroviralen Expressionsverktors

Als Grundlage für die Klonierungen diente der Vektor SFβ1 PSNEBH, der zur Familie der FMEV-Vektoren gehört (PCT/EP 95/03175).

Zunächst wurden die residualen *gag*-Anteile von SFβ1 PSNEBH über PCR-Klonierungsstrategien entfernt. Zu diesem Zweck wurde eine PCR mit den Primern ΔGAG+ (SEQ ID NO: 3) und ΔGAG/Not- (SEQ ID NO: 4) folgendermaßen durchgeführt:

Reaktionsgemisch:

10 ng SFβ1 PSNEBH
50 pmol Primer ΔGAG+
50 pmol Primer ΔGAG/Not-
80 μmol/l dNTP
10 μl 10x Pfu-PCR-Puffer
1 μl rekombinante Pfu-DNA-Polymerase
(Stratagene GmbH, Heidelberg)
ad 100 μl $H_2O$

Das Reaktionsgemisch wurde zunächst bei 95°C für 5 min inkubiert. Anschließend wurden 30 Cyclen unter folgenden Bedingungen durchgeführt:

95°C 30 s
55°C 40 s
72°C 2 min

Anschließend wurde das Reaktionsgemisch bei 72°C für 10 min inkubiert.

Das entstehende Produkt (ΔGAGNOT) wurde mittels Adenosintriphosphat phosphoryliert (s. Ausubel et al. 1994, a.a.O.) und in das mit EcoRV verdaute, dephosphorylierte Plasmid Bluescript pKS(+) (s. Ausubel et al. 1994, a. a.O.) subkloniert. Anschließend wurde das BglII/NotI-Fragment von ΔGAGNOT über BglII und NotI aus dem Klonierungsvektor herausgeschnitten und in den über BglII und NotI geöffneten SFβ1 PSNEBH inseriert.

Hieraus resultierte der Basisvektor SFβ11 NEBH, der anstelle des Transgens der Wahl eine multiple cloning site trägt.

**Beispiel 2**

Klonierung eines Spleißakzeptorsignales in SFβ11

Die Sequenz des Spleißakzeptorsignaless wurde dem FMEV-Vektor SF1MSN (M. Hildinger et al. (1998) Hum. Gene Ther., 9:33-42 entnommen, wobei die Consensus-Sequenz der sogenannten *branch site* leicht modifiziert wurde.

Es wurden 2 komplementäre Oligonucleotide synthetisiert, flankiert von einer SalI- (5') und NotI- (3') -Schnittstelle, mit folgenden Sequenzen:

SA-Oligo(+): SEQ ID NO: 6
SA-Oligo(-): SEQ ID NO: 7

Es erfolgte ein *annealing* der beiden Oligonucleotide unter folgenden Bedingungen (s. Ausubel et al. 1994, a.a.O.):

Reaktionsansatz

20 µl SA-Oligo(+) (50 pmol/µl)
20 µl SA-Oligo(-) (50 pmol/µl)
2,5 µl 4 mol/l NaCl
ad 100 µl TE (10 mM Tris·HCl, 1 mM EDTA) (pH 8,0 )

Dieser Ansatz wurde für 10 min bei 95°C inkubiert und anschließend langsam auf Raumtemperatur abgekühlt.

Zur Darstellung des Leaderfragments wurde eine PCR mit den Primern ΔGAG+ (SEQ ID NO: 3) und ΔGAG/Xho- (SEQ ID NO: 5) folgendermaßen durchgeführt:

Reaktionsgemisch

10 ng SFβ1 PSNEBH
50 pmol Primer ΔGAG+
50 pmol Primer ΔGAG/Xho-
80 µmol/l dNTP
10 µl 10x Pfu-PCR-Puffer
1 µl rekombinante Pfu-DNA-Polymerase
(Stratagene GmbH, Heidelberg)
ad 100 µl $H_2O$

Das Reaktionsgemisch wurde zunächst bei 95°C für 5 min inkubiert. Anschließend wurden 30 Cyclen unter folgenden Bedingungen durchgeführt:

95°C 30 s
55°C 40 s
72°C 2 min

Anschließend wurde das Reaktionsgemisch bei 72°C für 10 min inkubiert.

Das entstehende Produkt (ΔGAGXHO) wurde mittels Adenosintriphosphat phosphoryliert (s. Ausubel et al. 1994, a.a.O.) und in das mit EcoRV verdaute, dephosphorylierte Plasmid Bluescript pKS(+) (s. Ausubel et al. 1994, a. a.O.) subkloniert. Anschließend wurde das BglII/XhoI-Fragment über BglII und XhoI aus dem Klonierungsvektor herausgeschnitten.

Anschließend erfolgte eine 3-Fragment-Ligation von ΔGAGXHO (verdaut mit BglII und XhoI) mit dem annealten, doppelsträngigen Spleißakzeptor-Oligonucleotid (mit den Schnittstellen SalI und NotI) in den über BglII und NotI geöffneten Basisvektor SFβ1 PSNEBH.

Hieraus resultierte der Basisvektor SFβ71 NEBH.

**Beispiel 3**

Site directed mutagenesis von AUG-Stellen

Im 5'-untranslatierten Bereich des SFβ71 NEBH befindet sich noch ein ATG-Startcodon. Dieses wurde unter Verwendung einer PCR-gestützten *site-directed mutagenesis* (QuikChange *site-directed mutagenesis* Kit, Stratagene GmbH, Heidelberg, Vorgehen nach Herstellerprotokoll) eliminiert. Zu diesem Zweck wurde eine PCR mit den Primern MUT/Leader-ATG/+ (SEQ ID NO: 8) und MUT/Leader-ATG/- (SEQ ID NO: 9) folgendermaßen durchgeführt:

Reaktionsgemisch:

100 ng SFβ71 NEBH
10 pmol Primer MUT/Leader-ATG/+
10 pmol Primer MUT/Leader-ATG/-
80 μmol/l dNTP
5 μl 10x Pfu-PCR-Puffer
1 μl native Pfu-DNA-Polymerase
(Stratagene GmbH, Heidelberg)
ad 50 μl $H_2O$

Das Reaktionsgemisch wurde zunächst bei 95°C für 5 min inkubiert. Anschließend wurden 30 Cyclen unter folgenden Bedingungen durchgeführt:

95°C 30 s
55°C 60 s
68°C 10 min

Anschließend wurde das Reaktionsgemisch bei 68°C für 20 min inkubiert.
Das Reaktionsprodukt wurde anschließend eine Stunde mit DpnI verdaut und in Bakterien des Stammes CMK mittels der Calciumchlorid-Methode transformiert (s. Ausubel et al. 1994, a.a.O.). Der Erfolg der Mutagenese wurde mittels Sequenzierung verifiziert.
Hieraus resultierte der Basisvektor SFβ91 NEBH.

**Beispiel 4**

Site directed mutagenesis von kryptischen Spleißakzeptorstellen, Spleißdonorstellen und poly-A-Stellen

Kryptische Spleißakzeptorstellen, Spleißdonorstellen und poly-A-Stellen in der mdr-1 cDNA (Chen et al., Cell 47:381-389 (1986), Sequenz erhältlich über EMBL Accession Number M 14758) wurden mittels site directed mutagenesis (siehe oben) unter Einfügung stiller Mutationen (d.h. unter Wahrung der Wildtyp-Aminosäuresequenz) entfernt.
Nachfolgend sind für das unten beschriebene experimentelle Vorgehen immer das erste Oligonikleotid Primer A und das zweite Oligonikleotid Primer B.
Das Spleißdonor-Konsensussignal SD1 (Position +339 der MDR1 cDNA, Mutation G zu A) wurde unter Verwendung des Quikchange-site directed mutagnesis kit (Stratagene) sowie der Doppelstrangoligonukleotide SD1+ (SEQ ID NO: 10) und SD1- (SEQ ID NO: 11) mutiert.
Das Spleißakzeptor-Konsensussignal SA1 (Position +2320 der MDR1 cDNA, Mutation G zu A) wurde unter Verwendung des Quikchange-site directed mutagnesis kit (Stratagene) sowie der Doppelstrangoligonukleotides SA1+ (SEQ ID NO: 12) und SA1- (SEQ ID NO: 13) mutiert. Es resultierte die Variante cDNA mSA1.
Das Polyadenylierungs-Konsensussignal AATAAA1 (Position +3303 der MDR1 cDNA, Mutation A zu T) wurde unter Verwendung des Quikchange-site directed mutagnesis kit (Stratagene) sowie der Doppelstrangoligonukleotide AATAAA + (SEQ ID NO: 14) und AATAAA - (SEQ ID NO: 15) mutiert.
Alle Mutagenesen wurden (abgesehen von den jeweils eingesetzten Primerpaaren) unter identischen Bedingungen gemäß den Herstellerangaben (QuikChange *site-directed mutagenesis* Kit, Stratagene GmbH, Heidelberg) durchgeführt, wobei ein von pKS(+) abgeleitetes Plasmid eingesetzt wurde (pKS m1), das den kodierenden Bereich von mdr1 enthielt:

Reaktionsgemisch:

100 ng pKS m1
10 pmol Primer A
10 pmol Primer B
80 µmol/l dNTP
5 µl 10x Pfu-PCR-Puffer
1 µl native Pfu-DNA-Polymerase
ad 50 µl $H_2O$

Das Reaktionsgemisch wurde zunächst bei 95°C für 5 min inkubiert. Anschließend wurden 30 Cyclen unter folgenden Bedingungen durchgeführt:

95°C 30 s
55°C 60 s
68°C 10 min

Anschließend wurde das Reaktionsgemisch bei 68°C für 20 min inkubiert.

Die alle diese Modifikationen enthaltene MDR1 cDNA wurde als "m4" bezeichnet. Durch Klonierung der m4 cDNA in den Basisvektor SFβ71 und durch Klonierung der mSA1 cDNA in den Basisvektor SFβ91 wurden die Konstrukte SFβ71m4 und SFβ91mSA1 (s. Abb. 2) geschaffen, die ein im retroviralen Kontext stabiles MDR1 Gen übertragen und exprimieren. Diese Konstrukte können als Grundlage dienen für bi- und oligocistronische MDR1 Vektoren, unabhängig von der Wahl anderer regulatorischer Elemente.

**Beispiel 5**

Bestimmung des Titers des retroviralen Expressionsvektors

Als Indikatorgen wurde die menschliche MDR1 cDNA verwendet. MDR1 cDNA wurde mittels Standardmethoden (Ausubel et al., a.a.O.) in den Vektor SFβ11 NEBH und SFβ71 NEBH kloniert. Hierfür wurde der 5' untranslatierte Anteil der cDNA über PCR gerichtete Klonierung exakt deletiert und das Startkodon der MDR1-cDNA mit einem Kozak-Konsensus zur Optimierung des Translationsstartes sowie einer vorangeschalteten Restriktionsstelle für das Enzym NotI versehen. Es resultierten die MDR1 tragenden Derivate SFβ11m1 und SFβ71m1.

Zur Analyse der Eigenschaften von SFβ11m1 und SFβ71m1 bezüglich Titer und Translationseffizienz wurden Vergleichsvektoren kloniert, die ebenfalls MDR1 unter identischer FMEV-Enhancerkontrolle, aber mit konventionellen untranslatierten Sequenzen des Vektors tragen. Es handelt sich um SFβ1m1 (LX-Typ), SFβ6m1 (MFG-Typ) oder SF7m (LX-Typ mit Spleißakzeptor Oligonukleotid) (siehe Figur 3a).

Am Vortag wurde eine konfluente 9-cm-Schale eines Virusproduzenten (GP&AM12 American Typ Culture Collection (ATCC CRL-9641)) mit 5 ml frischem Medium (IMDM + 10% hitzeinaktiviertes fötalem Kälberserum (FCS)) gefüttert. Parallel wurden 10 000 HT1080 (humane Fibrosarkomzellen, ATCC CRL-9641) in jeweils eine Vertiefung einer 24-Loch-Platte eingesät. Am nächsten Tag wurde der virushaltige Überstand durch ein 1,2 µm-Filter sterilfiltriert und serielle 1:5-Verdünnungen in IMDM + 10% hitzeinaktiviertem FCS + 4 µg/ml Polybrene® (Hexadimethrinbromid, Copolymer aus N,N,N',N'-Tetramethyl-1,6-hexandiamin und 1,3-Dibrompropan; Polykation zur besseren Wechselwirkung zwischen Virus und Zelle bei der Injektion) angesetzt. Daraufhin wurde das Medium der 24-Loch-Platte entfernt und in Dreifachbestimmung 1 ml virushaltiger Überstand der entsprechenden Verdünnungsstufe aufgelegt gemäß folgendem Schema:

| | | | | | |
|---|---|---|---|---|---|
| 1:5 | 1:5 | 1:5 | 1:3125 | 1:3125 | 1:3125 |
| 1:25 | 1:25 | 1:25 | 1:5625 | 1:5625 | 1:5625 |
| 1:125 | 1:125 | 1:125 | 1:78125 | 1:78125 | 1:78125 |
| 1:625 | 1:625 | 1:625 | kein Virus | kein Virus | kein Virus |

Am folgenden Tag wurde erneut das Medium der 24-Loch-Platte entfernt und die Zellen mit IMDM + 10% FCS + 20 ng/ml Colchicin gefüttert. Dies wurde alle 2 Tage wiederholt, bis keine lebenden Zellen sich in den Vertiefungen ohne Virusüberstand mehr befanden.

Der Titer ergibt sich durch Multiplikation der Anzahl der Klone in einer bestimmten Vertiefung mit der entsprechenden Verdünnungsstufe. Befindet sich zum Beispiel bei der 1:78125-Verdünnung im Durchschnitt ein Klon in jeder Vertiefung, so betrüge der Gesamttiter 78125.

In diesen Experimenten zeigte es sich, daß die erfindungsgemäßen Vektoren in hohen Titern produziert werden können, wobei bis zu $5 \cdot 10^6$ infektiöse Einheiten pro ml erreicht werden können.

**Beispiel 6**

Untersuchung der Translationseffizienz

Zur Untersuchung der Translationseffezienz der erfindungsgemäßen Vektoren wurden menschliche K562 Erythroleukämizellen mit Überständen Vektor-produzierender GP&envAM12-Zellen (ATCC CRL-9641) infiziert und polyklonale Massenkulturen durch milde, aber hintergrundfreie Selektion in 5 ng Colchicin/ml Zellkulturmedium angelegt. K562-Zellen, die mit retroviralen Vektoren ohne die MDR1-cDNA transfiziert werden, sterben unter diesen Bedingungen. Die Massenkulturen wurden anschließend gegen steigende Dosen von Colchicin im Weichagarmedium auskloniert.

Dafür wurde zuerst IMDM + 10% FCS und 0,33% Bacto-Agar angesetzt. Anschließend wurde das Medium durch Zugabe von Colchicin auf

a) 20 ng/ml Colchicin
b) 40 ng/ml Colchicin
c) 60 ng/ml Colchicin

Endkonzentration eingestellt. Daraufhin wurden 500 K562 pro ml Medium zugegeben und jeweils 1 ml in eine Vertiefung einer 12-Loch-Platte pipettiert und zwar nach folgendem Schema:

| | | |
|---|---|---|
| 500 K562 0 ng/ml Colchicin | 500 K562 0 ng/ml Colchicin | 500 K562 0 ng/ml Colchicin |
| 500 K562 20 ng/ml Colchicin | 500 K562 20 ng/ml Colchicin | 500 K562 20 ng/ml Colchicin |
| 500 K562 40 ng/ml Colchicin | 500 K562 40 ng/ml Colchicin | 500 K562 40 ng/ml Colchicin |
| 500 K562 60 ng/ml Colchicin | 500 K562 60 ng/ml Colchicin | 500 K562 60 ng/ml Colchicin |

Nach kurzer Inkubation bei 4°C (Festwerden des Agars) wurden die Platten für 14 Tage bei 37°C und 5% $CO_2$ inkubiert. Nach 14 Tagen wurde die Anzahl der lebenden Kolonien pro Vertiefung bestimmt. Die relative Klonierungseffizienz ergibt sich durch Division der durchschnittlichen Anzahl der Klone bei einer bestimmten Colchicin-Konzentration durch die durchschnittliche Anzahl der Klone ohne Colchicinzugabe.

Die Klonierungseffizienz in Gegenwart hoher Colchicindosen ist ein sehr sensitiver Indikator des Expressionsniveaus von MDR1 [C. Baum et al. (1995) J. Virol. 69:7541-7547]. Die erfindungsgemäßen Konstrukte (SFβ11m1 und SFβ71m1) erzielten eine signifikante (2,5fache) Erhöhung der Klonierungseffizienz gegenüber dem LX-Typ Standardvektor (SFβ1m1), dessen Aktivität als 1 gesetzt wurde (siehe Figur 3B). Die erfindungsgemäßen Vektoren waren auch der im MFG-Vektor realisierten Positionierung anstelle des viralen *env*-Gens (SFβ6m1) oder der SFβ1m1-Variante mit Spleißakzeptoroligonukleotid (SFβ7m1) überlegen. In diesen Experimenten zeigte sich, daß die erfindungsgemäßen Vektoren eine verbesserte Genexpression vermitteln. Dies beruht wahrscheinlich auf erhöhter posttranskriptioneller Effizienz (Erhöhung der Translationseffizienz), da die verwendeten Vektoren bezüglich der transkriptionellen Kontrollregionen identisch waren.

**Beispiel 7**

Expression von humanized enhanced green flourescent protein mittels den erfindungsgemäßen retroviralen Vektoren

In die erfindungsgemäßen Vektoren SFβ1, SFβ6, SFβ71 und SFβ91 wurde das Transgen *humanized enhanced green fluorescent protein* (abgekürzt als eGFP; CLONTECH GmbH, Heidelberg) kloniert. Die Expression des Transgens in der erythroleukämischen Zellinie K562 wurde durchflußcytometrisch analysiert.

a) Klonierung von SFβ1eGFP / SFβ6eGFP / SFβ71eGFP / SFβ91eGFP

eGFP wurde über PCR mit dem Vektor pEGFP-C1 (CLONTECH GmbH, Heidelberg) als Matrize amplifiziert unter Verwendung der Primer eGFP+ (SEQ ID NO: 16) und eGFP- (SEQ ID NO: 17):

Reaktionsgemisch:

10 ng pEGFP-C1
50 pmol Primer eGFP
50 pmol Primer eGFP
80 µmol/l dNTP
10 µl 10x Pfu-PCR-Puffer
1 µl rekombinante Pfu-DNA-Polymerase
(Stratagene GmbH, Heidelberg)
ad 100 µl $H_2O$

Das Reaktionsgemisch wurde zunächst bei 95°C für 5 min inkubiert. Anschließend wurden 30 Cyclen unter folgenden Bedingungen durchgeführt:

95°C 30 s
55°C 40 s
72°C 2 min

Anschließend wurde das Reaktionsgemisch bei 72°C für 10 min inkubiert.

Das entstehende Produkt (eGFP) wurde mittels Adenosintriphosphat phosphoryliert (s. Ausubel et al. 1994, a.a.O.) und in das mit EcoRV verdaute, dephosphorylierte Plasmid Bluescript pKS(+) (s. Ausubel et al. 1994, a.a.O.) subkloniert. Anschließend wurde eGFP über NotI und EcoRI aus dem Klonierungsvektor herausgeschnitten und in die Basisvektoren, die jeweils über einen Restriktionsverdau mit den Enzymen NotI und EcoRI liniearisiert und anschließend mittels Alkalischer Phosphatase vom Rind dephosphoryliert (s. Ausubel et al. 1994, a.a.O.) wurden, inseriert. Hieraus resultierten die Vektoren SFβ1eGFP / SFβ6eGFP / SFβ71eGFP / SFβ91eGFP.

b) Datenerhebung

Die amphotrope Verpackungszellinie GP&Am12 wurde mit 10 µg Plasmid-DNA von SFβ1eGFP, SFβ6eGFP, SFβ71eGFP bzw. SFβ91eGFP mittels Calciumphosphat-Präzipitation (s. Ausubel et al. 1994. Current protocols in molecular biology. John Wiley & Sons. New York, N. Y., USA) transfiziert. Ein Tag nach der Transfektion wurde virushaltiger Überstand geerntet und K562-Zellen infiziert. Nach weiteren drei Tagen wurde die eGFP-Expression der K562 durchflußcytometrisch mit einem FACScalibur (Becton Dickinson GmbH, Heidelberg) analysiert und die mittlere Fluoreszenz der exprimierenden K562 ermittelt. Es ergaben sich die in Figur 4 gezeigten Werte (bezogen auf SFβ1eGFP).

**Beispiel 8**

Nachweis der Stabilität der modifizierten MDR1-cDNA

Der Nachweis der Stabilität der modifizierten MDR1-cDNAs "mSA1" und "m4" erfolgte über Durchflußzytometrie von K562-Massenkulturen, die retroviral mit MDR1-Vektoren transduziert wurden, ohne zuvor auf Intaktheit der MDR1-cDNA zu selektieren. Hierzu wurde in SFβ71m4 und SFβ91mSA1 eine Genkassette eingesetzt, die 3' der MDR1-cDNA das Neomycin-Resistenzgen (neoR) unter Kontrolle eines retroviralen Spleißakzeptors (abgeleitet von SF1MSN [M. Hildinger et al., a.a.O.] trägt. Die resultierenden Vektoren wurden als SFβ91mSA1SN und SFβ71m4SN bezeichnet. Die Selektion transduzierter Zellen erfolgte mit G418 (Geneticin), gegen das neoR Resistenz vermittelt.

Für den Rh123-Efflux-assay wurden $10^6$ K562 in 2 ml Medium mit 10 µg/ml Rh123 für 30 min bei 37°C inkubiert. Nach zweimaligem Waschen mit jeweils 5 ml PBS wurden die Zellen für weitere 2 Stunden in 2 ml Medium bei 37°C inkubiert. In dieser Zeit erfolgt das Ausschleusen von Rh123 aus den Zellen. Nach zwei weiteren Waschschritten wurden die Zellen in 1 ml PBS aufgenommen und an einem FACS-Analyzer der Firma Becton-Dickinson (FACSCalibur) analysiert, wobei die Rh123-Fluoreszenz auf dem Fluoreszenz-Kanal 1 gemessen wurde. Sofern die Zellen MDR1 exprimieren, wird Rh123 aus den Zellen ausgeschleust, die Fluoreszenz der Zellen ist dementsprechend geringer.

Im Rahmen dieser Experimente wurde deutlich, daß bei Transduktion mit SFβ91mSA1SN oder SFβ71m4SN wenige oder keine MDR1-defekten Zellen auftreten, während bei Transduktion mit SFβ1mSN ca 40 % MDR1-defekte Zellen zu verzeichnen sind (siehe Figur 5). Diese Defekte beruhen auf genetischen Rearrangements der cDNA, die durch die Eliminierung der Prozessierungssignale in der m4- bzw. mSA1-Variante unterdrückt sind.

**Kurze Beschreibung der Figuren**

Figur 1

Vergleich retroviraler Vektoren des Typs LX und MFG mit der neuen Form.

LX und MFG enthalten die gag+-Sequenzen (*gag*Δ), MFG zusätzlich noch Teile von pol (*pol*Δ). Die Transkriptionskontrolle erfolgt vom LTR, insbesondere von der Enhancer/Promotorsequenz in der U3-Region. Die Translation des Transgens erfolgt bevorzugt über gespleißte Varianten der Vektor-RNA, die durch Erkennung der Spleißdonor- (SD) und Spleißakzeptor- (SA) Stellen entstehen. Der Pfeil über der symbolisch dargestellten Vektor-DNA zeigt die Vektor-RNA an, die gestrichelten Anteile beziehen sich auf die durch Spleißen entfernten Anteile des Transkriptes. Das Transgen (cDNA) beginnt beim LX-Vektor inmitten der *gag*-Region, alle übrigen Virus-kodierenden Sequenzen sind eliminiert. Beim MFG-Vektor vorhandene Teile der pol-Sequenzen enthalten den Spleißakzeptor des env-Gens. In beiden Fällen (LX und MFG) ist das Startkodon des *gag*-Gens zerstört (durchgestrichenes AUG), es finden sich jedoch multiple weitere AUGs in allen Leserahmen stromaufwärts des Transgens.

Bei der besonders bevorzugten Ausführungsform des erfindungsgemäßen Vektors sind alle Virus-kodierenden Sequenzen eliminiert. Der Vektor wurde daher als *gag-pol-env* deletierten Vektor (GDV) bezeichnet. Es sind nur noch cis-regulatorische Anteile des Virus vorhanden (LTRs mit U3, R und U5 sowie die Verpackungsregion (Ψ).

Figur 2

Erfindungsgemäße Vektoren SFβ91mSA1 und SFβ71m4, schematische Abbildung der proviralen Form. Die Vektoren enthalten keine kodierenden Sequenzen des Retrovirus. An der CAP-STelle beginnt die Transkription der viralen RNA. Das Plasmid-Rückgrat stammt vom Klonierungsvektor pUC19 (New England Biolabs) und hat keinen Einfluß auf die Funktion des retro viralen Vektors.

Figur 3

Einfluß des neuen Vektoraufbaus (GDV-Typ) auf die Stärke der Genexpression, im vorliegenden Beispiel: Verbesserung der Vektorvermittelten Multidrug-Resistenz im Hochdosisbereich.

A: Vektoraufbau. SD: Spleißdonor des Vektors; *gag*: *gag*-Genfragment; SA*pol*: Spleißakzeptor-enthaltendes *pol*-Fragment, SAO: Spleißakzeptoroligonukleotid

B: Klonierungseffizienz (Mittelwert von 3 Versuchen, Standardabweichungen waren geringer als 25 %)

Figur 4

Einfluß des neuen Vektoraufbaus (GDV-Typ) auf die Stärke der Genexpression, im vorliegenden Beispiel: Erhöhung der eGFP-Fluoreszenz in transduzierten Zellen.

A: Vektoraufbau,
B: Mittlere Fluoreszenz (Mittelwert von mehr als 10 000 Ereignissen)

Figur 5

Bereinigung der MDR1-cDNA von aberrantem Spleißen

A: Vektoren, mit denen K562-Zellen transduziert wurden gefolgt von Selektion mit G418 (Resistenz über das neoR-Gen des Vektors).

B: Rhodamin 123-Efflux-Versuch, mit dem die Integrität der mdr1-cDNA in der G418-selektierten Massenkultur nachgewiesen wird (Hildinger et al. (1997) Hum. Gene Ther. 9:33-42).
Bei SFβ91mSA1SN ist die mdr1-cDNA partiell, bei SFβ71m4SN vollständig intakt.
Dies zeigt, daß das interne Spleißen der mdr1-cDNA vor der Reversen Transkription verhindert wird. Außerdem zeigt sich die erhöhte Expression von mdr1 im weiter zur niedrigen Fluoreszenz verschobenen Gipfel des Histogramms.

**Patentansprüche**

1. Retroviraler Vektor, der eine Nukleotidsequenz N der allgemeinen Formel

5'-Ende -- [5'-UTR] - [gag] - [ex] - [vir] -- 3'-Ende

enthält, wobei
[5'-UTR] eine nicht-kodierende Nukleotidsequenz darstellt, die in Retroviren unmittelbar 5', d.h., stromaufwärts des Startcodons des *gag*-Genes liegt,
[gag] einen Abschnitt der Nukleotidsequenz darstellt, die für das *gag*-Gen kodiert,
[ex] eine nicht-virale Nukleotidsequenz darstellt und
[vir] eine retrovirale Nukleotidsequenz darstellt,
**dadurch gekennzeichnet,**
**daß** die Anzahl an Nukleotiden in [gag] weniger als 400 bp umfaßt.

2. Vektor nach Anspruch 1, **dadurch gekennzeichnet, daß** die Anzahl an Nukleotiden in [gag] weniger als 200 bp, vorzugsweise weniger als 80 bp umfaßt.

3. Vektor nach den Ansprüchen 1 oder 2, **dadurch gekennzeichnet, daß** [gag] keine Nukleotide umfaßt.

4. Vektor nach den Ansprüchen 1 bis 3, **dadurch gekennzeichnet, daß** er keine für das Pro-Protein, das Env-Protein, das Pol-Protein und/oder andere virale Proteine kodierende Sequenzen oder Sequenzabschnitte enthält.

5. Vektor nach den Ansprüchen 1 bis 4, **dadurch gekennzeichnet, daß** die Nukleotidsequenz [ex] mindestens eine Nukleotidsequenz, die für ein nicht-virales Protein kodiert, oder eine nicht-translatierte, nicht-virale RNA enthält.

6. Vektor nach Anspruch 5, **dadurch gekennzeichnet, daß** das erste Nukleotid des Startcodons der Nukleotidsequenz, die für ein nicht-virales Protein codiert, das 5'-Ende der Nukleotidsequenz [ex] ist.

7. Vektor nach den Ansprüchen 1 bis 6, **dadurch gekennzeichnet, daß** die Nukleotidsequenz [ex] zusätzlich regulatorische Nukleotidsequenzen umfaßt.

8. Vektor nach Anspruch 7, **dadurch gekennzeichnet, daß** die regulatorischen Nukleotidsequenzen Spleißakzeptorstellen, Promotor- und/oder Enhancersequenzen sind.

9. Vektor nach den Ansprüchen 1 bis 8, **dadurch gekennzeichnet, daß** [5'-UTR] mindestens einen Sequenzabschnitt enthält, der einen Spleißakzeptor darstellt.

10. Vektor nach Anspruch 9, **dadurch gekennzeichnet, daß** der Spleißakzeptor ein Oligonukleotid mit einer Länge von 10 bis 150 bp ist.

11. Vektor nach den Ansprüchen 1 bis 10, **dadurch gekennzeichnet, daß** [5'-UTR] kein Nukleotid-Triplett mit der Sequenz ATG enthält.

12. Vektor nach den Ansprüchen 5 bis 11, **dadurch gekennzeichnet, daß** die in der Nukleotidsequenz [ex] für nicht-virale Proteine kodierenden Nukleotidsequenzen keine kryptischen Spleißdonorstellen, Spleißakzeptorstellen und/oder kryptischen poly-A-Stellen enthalten.

13. Vektor nach den Ansprüchen 1 bis 12, **dadurch gekennzeichnet, daß** 5' von [ex] eine Leaderregion liegt.

14. Vektor nach Anspruch 13, **dadurch gekennzeichnet, daß** die Leaderregion aus MESV stammt.

15. Vektor nach den Ansprüchen 1 bis 14, **dadurch gekennzeichnet, daß** 3' von [vir] ein 3'-LTR liegt.

16. Vektor nach Anspruch 15, **dadurch gekennzeichnet, daß** der 3'-LTR aus dem spleen focus forming virus (SFFVp) stammt.

17. Vektor nach den Ansprüchen 1 bis 16, **dadurch gekennzeichnet, daß** die in der Nukleotidsequenz [ex] liegende Nukleotidsequenz, für das MDR1-Protein kodiert.

18. Vektor nach Anspruch 17, entsprechend DSM 12065 oder DSM 12066.

**19.** Vektor nach Anspruch 17, **dadurch gekennzeichnet, daß** er dem unter DSM 12065 hinterlegten Vektor entspricht, wobei er

a) keine Nukleotidsequenzen enthält, die für virale Proteine kodieren,

b) am 5'-Ende der proviralen DNA die U3-, R- und U5-Regionen des 5'-LTR enthält,

c) im 5'-LTR die CAP-Stelle liegt, an der die Transkription der viralen mRNA beginnt,

d) 3' vom 5'-LTR eine Nukleotidsequenz liegt, die einen Spleißakzeptor, eine Verpackungsregion (ψ) und einen Spleißdonor enthält, wobei das Nukleotid 271 (gezählt ab CAP-Stelle) mutiert ist, um ein kryptisches AUG zu entfernen,

e) an der Stelle, an der sich die für das *gag*-Gen kodierenden Sequenzen befanden, die Variante mSA1 des MDR1 Gens enthält, wobei an Position 2320 (gezählt ab Startcodon des MDR1-Gens) ein kryptischer Spleißakzeptor mutiert ist,
und

f) sich 3' der MDR1-Variante mSA1 der 3'-LTR mit den U3-, R- und U5-Regionen befindet.

**20.** Vektor nach Anspruch 17, **dadurch gekennzeichnet, daß** er dem unter DSM 12066 hinterlegten Vektor entspricht, wobei er

a) keine Nukleotidsequenzen enthält, die für virale Proteine kodieren,

b) am 5'-Ende der proviralen DNA die U3-, R- und U5-Regionen des 5'-LTR enthält,

c) im 5'-LTR die CAP-Stelle liegt, an der die Transkription der viralen RNA beginnt,

d) 3' vom 5'-LTR eine Nukleotidsequenz liegt, die einen Spleißakzeptor, eine Verpackungsregion (ψ) und einen Spleißdonor enthält,

e) an der Stelle, an der sich die für das *gag*-Gen kodierenden Sequenzen befanden, die Variante m4 des MDR1 Gens enthält, wobei an Position 339 (gezählt ab Startcodon des MDR1-Gens) ein kryptischer Spleißdonor, an Position 2320 (gezählt ab Startcodon des MDR1-Gens) ein kryptischer Spleißakzeptor und an Position 3303 (gezählt ab Startcodon des MDR1-Gens) ein kryptisches Poly(A)-Signal mutiert ist,
und

f) sich 3' der MDR1-Variante m4 der 3'-LTR mit den U3-, R- und U5-Regionen befindet.

**21.** Vektor nach Anspruch 20, **dadurch gekennzeichnet, daß** er zusätzlich an Position 271 (gezählt ab CAP-Stelle) ein mutiertes kryptisches AUG enthält.

**22.** Verfahren zur Gewinnung eines infektiösen Viruspartikels, **dadurch gekennzeichnet, daß** man eine verpackungskompetente Helferzelle mit einem retroviralen Vektor nach den Ansprüchen 1 bis 21 transfiziert und die Helferzelle in einem geeigneten Medium unter Bedingungen kultiviert, die für die Abgabe infektiöser Viruspartikel, die als Genom den retroviralen Vektor nach den Ansprüchen 1 bis 21 enthalten, geeignet sind.

**23.** Infektiöses Viruspartikel, **dadurch gekennzeichnet, daß** das Partikel den retroviralen Vektor nach den Ansprüchen 1 bis 21 enthält.

**24.** Wirtszelle, **dadurch gekennzeichnet, daß** sie mit dem retroviralen Vektor nach den Ansprüchen 1 bis 21 transfiziert ist.

**25.** Wirtszelle nach Anspruch 24, **dadurch gekennzeichnet, daß** sie mit einem infektiösen Viruspartikel nach Anspruch 23 infiziert ist.

**26.** Wirtszelle nach Anspruch 24, **dadurch gekennzeichnet, daß** sie mit einem Vektor entsprechend DSM 12065 oder DSM 12066 transfiziert ist.

**27.** Verwendung in vitro des retroviralen Vektors nach den Ansprüchen 1 bis 21 in der Gentherapie.

**28.** Verwendung des retroviralen Vektors nach den Ansprüchen 1 bis 21 zur Klonierung von Genen.

**29.** Verwendung des retroviralen Vektors nach den Ansprüchen 1 bis 21 zur Expression und/ oder Überexpression von Proteinen oder RNA.

**30.** Verwendung des retroviralen Vektors nach den Ansprüchen 1 bis 21 zur Transfektion hämatopoietischer Stammzellen.

**31.** Verfahren zur Gewinnung von Proteinen, **dadurch gekennzeichnet, daß** man eine Wirtszelle nach den Ansprüchen 24 bis 26 in einem geeigneten Medium unter Bedingungen kultiviert, die zur Expression der nicht-viralen Proteine, für die Nukleotidsequenzen in [ex] kodieren, notwendig sind und daß man das so erzeugte Protein von den Zellen und dem Medium abtrennt.

**32.** Verwendung des retroviralen Vektors nach den Ansprüchen 1 bis 21 zur Herstellung eines pharmazutischen Präparates zur Gentherapie, **dadurch gekennzeichnet, daß** der Vektor in geeigneter Form vorliegt, um in die Zielzellen eingeführt zu werden.

**33.** Verwendung des retroviralen Vektors nach den Ansprüchen 18 bis 21 zur Herstellung eines pharmazeutischen Präparates für die Transfektion hämatopoietischer Stammzellen zur Erzielung einer Resistenz hämatopoietischer Stammzellen bei Chemotherapie.

**34.** Pharmazeutisches Präparat, **dadurch gekennzeichnet, daß** es einen Vektor nach den Ansprüchen 1 bis 21 zusammen mit pharmazeutisch verträglichen Hilfs- und/oder Trägerstoffen enthält.

## Claims

**1.** Retroviral vector, which comprises a nucleotide sequence N of the general formula

5' end -- [5' UTR] - [gag] - [ex] - [vir] -- 3' end,

where
[5' UTR] is a non-coding nucleotide sequence which, in retroviruses, is located immediately 5', i.e. upstream, of the start codon of the *gag* gene,
[gag] is a section of the nucleotide sequence, which codes for the *gag* gene,
[ex] is a non-viral nucleotide sequence and
[vir] is a retroviral nucleotide sequence,
**characterized in that**
the number of nucleotides in [gag] comprises less than 400 bp.

**2.** Vector according to Claim 1, **characterized in that** the number of nucleotides in [gag] comprises less than 200 bp, preferably less than 80 bp.

**3.** Vector according to Claim 1 or 2, **characterized in that** [gag] does not comprise any nucleotides.

**4.** Vector according to Claims 1 to 3, **characterized in that** it does not comprise any sequences or sequence sections coding for the Pro protein, the Env protein, the Pol protein and/or other viral proteins.

**5.** Vector according to Claims 1 to 4, **characterized in that** the nucleotide sequence [ex] comprises at least one nucleotide sequence coding for a non-viral protein or an untranslated, non-viral RNA.

**6.** Vector according to Claim 5, **characterized in that** the first nucleotide of the start codon of the nucleotide sequence coding for a non-viral protein is the 5' end of the nucleotide sequence [ex].

7. Vector according to Claims 1 to 6, **characterized in that** the nucleotide sequence [ex] additionally comprises regulatory nucleotide sequences.

8. Vector according to Claim 7, **characterized in that** the regulatory nucleotide sequences are splice acceptor sites, promoter and/or enhancer sequences.

9. Vector according to Claims 1 to 8, **characterized in that** [5' UTR] comprises at least one sequence section which is a splice acceptor.

10. Vector according to Claim 9, **characterized in that** the splice acceptor is an oligonucleotide of from 10 to 150 bp in length.

11. Vector according to Claims 1 to 10, **characterized in that** [5' UTR] does not comprise any nucleotide triplet having the sequence ATG.

12. Vector according to Claims 5 to 11, **characterized in that** the nucleotide sequences coding for non-viral proteins in the nucleotide sequence [ex] do not comprise any cryptic splice donor sites, splice acceptor sites and/or cryptic poly-A sites.

13. Vector according to Claims 1 to 12, **characterized in that** a leader region is located 5' of [ex].

14. Vector according to Claim 13, **characterized in that** the leader region is derived from MESV.

15. Vector according to Claims 1 to 14, **characterized in that** a 3' LTR is located 3' of [vir].

16. Vector according to Claim 15, **characterized in that** the 3' LTR is derived from the spleen focus forming virus (SFFVp).

17. Vector according to Claims 1 to 16, **characterized in that** the nucleotide sequence located within the nucleotide sequence [ex] codes for the MDR1 protein.

18. Vector according to Claim 17, corresponding to DSM 12065 or DSM 12066.

19. Vector according to Claim 17, **characterized in that** it corresponds to the vector deposited under DSM 12065, and

    a) does not comprise any nucleotide sequences coding for viral proteins,

    b) comprises, at the 5' end of the proviral DNA, the U3, R and U5 regions of the 5' LTR,

    c) the CAP site at which transcription of the viral mRNA initiates is located in the 5' LTR,

    d) a nucleotide sequence comprising a splice acceptor, a packaging region (p) and a splice donor is located 3' of the 5' LTR, the nucleotide 271 (as counted from the CAP site) being mutated in order to remove a cryptic AUG,

    e) comprises, at the site at which the sequences coding for the gag gene have been, the variant mSA1 of the MDR1 gene, a cryptic splice acceptor being mutated at position 2320 (as counted from the start codon of the MDR1 gene),
    and

    f) the 3' LTR containing the U3, R and U5 regions is 3' of the MDR1 variant mSA1.

20. Vector according to Claim 17, **characterized in that** it corresponds to the vector deposited under DSM 12066, and

    a) does not comprise any nucleotide sequences coding for viral proteins,

    b) comprises, at the 5' end of the proviral DNA, the U3, R and U5 regions of the 5' LTR,

c) the CAP site at which transcription of the viral RNA initiates is located in the 5' LTR,

d) a nucleotide sequence comprising a splice acceptor, a packaging region (p) and a splice donor is located 3' of the 5' LTR,

e) comprises, at the site at which the sequences coding for the *gag* gene have been, the variant m4 of the MDR1 gene, a cryptic splice donor being mutated at position 339 (as counted from the start codon of the MDR1 gene), a cryptic splice acceptor being mutated at position 2320 (as counted from the start codon of the MDR1 gene) and a cryptic poly(A) signal being mutated at position 3303 (as counted from the start codon of the MDR1 gene),
and

f) the 3' LTR containing the U3, R and U5 regions is 3' of the MDR1 variant m4.

**21.** Vector according to Claim 20, **characterized in that** it additionally comprises a mutated cryptic AUG at position 271 (as counted from the CAP site).

**22.** Process for obtaining an infectious virus particle, **characterized in that** a packaging-competent helper cell is transfected with a retroviral vector according to Claims 1 to 21 and the helper cell is cultured in a suitable medium under conditions suitable for the release of infectious virus particles whose genome is the retroviral vector according to Claims 1 to 21.

**23.** Infectious virus particle, **characterized in that** the particle comprises the retroviral vector according to Claims 1 to 21.

**24.** Host cell, **characterized in that** it has been transfected with the retroviral vector according to Claims 1 to 21.

**25.** Host cell according to Claim 24, **characterized in that** it has been infected with an infectious virus particle according to Claim 23.

**26.** Host cell according to Claim 24, **characterized in that** it has been transfected with a vector corresponding to DSM 12065 or DSM 12066.

**27.** Use IN VITRO of the retroviral vector according to Claims 1 to 21 in gene therapy.

**28.** Use of the retroviral vector according to Claims 1 to 21 for cloning genes.

**29.** Use of the retroviral vector according to Claims 1 to 21 for expression and/or overexpression of proteins or RNA.

**30.** Use of the retroviral vector according to Claims 1 to 21 for transfection of haematopoietic stem cells.

**31.** Process for obtaining proteins, **characterized in that** a host cell according to Claims 24 to 26 is cultured in a suitable medium under conditions necessary for expressing the non-viral proteins encoded by nucleotide sequences in [ex] and that the protein generated in this way is removed from the cells and the medium.

**32.** Use of the retroviral vector according to Claims 1 to 21 for preparing a pharmaceutical preparation for gene therapy, **characterized in that** the vector is present in a form suitable for being introduced into the target cells.

**33.** Use of the retroviral vector according to Claims 18 to 21 for preparing a pharmaceutical preparation for transfection of haematopoietic stem cells to achieve a resistance of haematopoietic stem cells in chemotherapy.

**34.** Pharmaceutical preparation, **characterized in that** it comprises a vector according to Claims 1 to 21 together with pharmaceutically compatible excipients and/or carriers.

**Revendications**

**1.** Vecteur rétroviral, qui contient une séquence nucléotidique N de formule générale

extrémité 5' -[5'-UTR]-[gag]-[ex]-[vir]- extrémité 3'

dans laquelle,
[5'-UTR] représente une séquence nucléotidique non codante, qui est située directement en 5', c'est-à-dire en amont du codon d'initiation du gène gag dans les rétrovirus,
[gag] représente un segment de la séquence nucléotidique, qui code pour le gène gag,
[ex] représente une séquence nucléotidique non virale et
[vir] représente une séquence nucléotidique rétrovirale,
**caractérisée en ce que**
le nombre de nucléotides dans [gag] est inférieur à 400 pb.

2. Vecteur selon la revendication 1, **caractérisé en ce que** le nombre de nucléotides dans [gag] est inférieur à 200 pb, de préférence inférieur à 80 pb.

3. Vecteur selon l'une des revendications 1 ou 2, **caractérisé en ce que** [gag] ne comprend aucun nucléotide.

4. Vecteur selon l'une des revendications 1 à 3, **caractérisé en ce qu'**il ne contient aucune séquence ou aucun segment codant pour la pro-protéine, la protéine Env, la protéine Pol et/ou d'autres protéines virales.

5. Vecteur selon l'une des revendications 1 à 4, **caractérisé en ce que** la séquence nucléotidique [ex] comprend au moins une séquence nucléotidique qui code pour une protéine non virale, ou qui contient un ARN non viral non traduit.

6. Vecteur selon la revendication 5, **caractérisé en ce que** le premier nucléotide du codon d'initiation de la séquence nucléotidique, qui code pour une protéine non virale, est l'extrémité 5' de la séquence nucléotidique [ex].

7. Vecteur selon l'une des revendications 1 à 6, **caractérisé en ce que** la séquence nucléotidique [ex] comprend en outre des séquences nucléotidiques régulatrices.

8. Vecteur selon la revendication 7, **caractérisé en ce que** les séquences nucléotidiques régulatrices sont des sites accepteurs d'épissage, des séquences de promotion et/ou d'amplification.

9. Vecteur selon l'une des revendications 1 à 8, **caractérisé en ce que** [5'-UTR] contient au moins un segment de séquence, qui représente un accepteur d'épissage.

10. Vecteur selon la revendication 9, **caractérisé en ce que** l'accepteur d'épissage est un oligonucléotide d'une longueur de 10 à 150 pb.

11. Vecteur selon l'une des revendications 1 à 10, **caractérisé en ce que** [5'-UTR] ne contient pas de triplette nucléotidique correspondant à la séquence ATG.

12. Vecteur selon l'une des revendications 5 à 11, **caractérisé en ce que** les séquences nucléotidiques codant pour la protéine non virale dans la séquence nucléotidique [ex] ne contiennent pas de sites donneurs d'épissage, de sites accepteurs d'épissage cryptés, et/ou de sites poly-A cryptiques.

13. Vecteur selon l'une des revendications 1 à 12, **caractérisé en ce qu'**une région leader est située en 5' de [ex].

14. Vecteur selon la revendication 13, **caractérisé en ce que** la région leader provient de MESV.

15. Vecteur selon l'une des revendications 1 à 14, **caractérisé en ce qu'**un 3'-LTR est situé en 3' de [vir].

16. Vecteur selon la revendication 15, **caractérisé en ce que** le 3'-LTR provient du SFFVp (spleen focus forming virus).

17. Vecteur selon l'une des revendications 1 à 16, **caractérisé en ce que** la séquence nucléotidique située dans la séquence nucléotidique [ex] code pour la protéine MDR1.

18. Vecteur selon la revendication 17, correspondant au dépôt DSM 12065 ou DSM 12066.

**19.** Vecteur selon la revendication 17, **caractérisé en ce qu'**il correspond au vecteur déposé sous la référence DSM 12065, et qui

a) ne contient aucune séquence nucléotidique, qui code pour des protéines virales,
b) contient à l'extrémité 5' de l'ADN proviral les régions U3, R et U5 du 5'-LTR,
c) porte un site CAP dans le 5'-LTR, à partir duquel commence la transcription de l'ARNm viral,
d) porte en 3' à une séquence nucléotidique partant du 5'-LTR, qui contient un accepteur d'épissage, une région d'encapsidation (Ψ) et un donneur d'épissage, le nucléotide 271 (en comptant à partir du site CAP) étant muté, afin d'éliminer un AUG cryptique,
e) contient, sur le site dans lequel se trouvaient les séquences codant pour le gène gag, la variante mSA1 du gène MDR1, un accepteur d'épissage cryptique étant muté en position 2320 (en comptant à partir du codon d'initiation du gène MDR1),
et
f) contient le 3'-LTR avec les régions U3, R et U5 en 3' de la variante mSA1 de MDR1.

**20.** Vecteur selon la revendication 17, **caractérisé en ce qu'**il correspond au vecteur déposé sous la référence DSM 12066, et qui

a) ne contient aucune séquence nucléotidique qui code pour des protéines virales,
b) contient à l'extrémité 5' de l'ADN proviral les régions U3, R et U5 de la 5'-LTR,
c) porte un site CAP dans le 5'-LTR, à partir duquel commence la transcription de l'ARN viral,
d) porte en 3' à une séquence nucléotidique partant du 5'-LTR, qui contient un accepteur d'épissage, une région d'encapsidation (Ψ) et un donneur d'épissage,
e) contient, sur le site dans lequel se trouvaient les séquences codant pour le gène gag, la variante m4 du gène MDR1, un donneur d'épissage cryptique étant muté en position 339 (en comptant à partir du codon d'initiation du gène MDR1), un accepteur d'épissage cryptique étant muté en position 2320 (en comptant à partir du codon d'initiation du gène MDR1) et un signal poly(A) cryptique étant muté en position 3303 (en comptant à partir du codon d'initiation du gène MDR1),
et
f) contient le 3'-LTR avec les régions U3, R et U5 en 3' de la variante m4 de MDR1.

**21.** Vecteur selon la revendication 20, **caractérisé en ce qu'**il contient en outre en position 271 (en comptant à partir du site CAP) un AUG crypté muté.

**22.** Procédé d'obtention d'une particule virale infectieuse, **caractérisé en ce qu'**on transfecte une cellule assistante compétente pour l'encapsidation avec un vecteur rétroviral selon l'une des revendications 1 à 21 et qu'on cultive la cellule auxiliaire dans un milieu adéquat dans des conditions qui sont adaptées pour la libération des particules virales infectieuses, qui contiennent le vecteur rétroviral comme génome selon les revendications 1 à 21.

**23.** Particule virale infectieuse, **caractérisée en ce que** la particule contient le vecteur rétroviral selon les revendications 1 à 21.

**24.** Cellule hôte, **caractérisée en ce qu'**elle est transfectée avec le vecteur rétroviral selon les revendications 1 à 21.

**25.** Cellule hôte selon la revendication 24, **caractérisée en ce qu'**elle est infectée avec une particule virale infectieuse selon la revendication 23.

**26.** Cellule hôte selon la revendication 24, **caractérisée en ce qu'**elle est transfectée par un vecteur correspondant aux références DSM 12065 ou DSM 12066.

**27.** Utilisation in vitro du vecteur rétroviral selon les revendications 1 à 21 en thérapie génique.

**28.** Utilisation du vecteur rétroviral selon les revendications 1 à 21 pour le clonage de gènes.

**29.** Utilisation du vecteur rétroviral selon les revendications 1 à 21 pour l'expression et/ou la surexpression de protéines ou d'ARN.

**30.** Utilisation du vecteur rétroviral selon les revendications 1 à 21 pour la transfection de cellules souches hématopoïétiques.

**31.** Procédé d'obtention de protéines, **caractérisé en ce qu'**on cultive une cellule hôte selon les revendications 24 à 26 dans un milieu adéquat dans des conditions, qui sont nécessaires à l'expression des protéines non virales, qui codent pour les séquences nucléotidiques dans [ex], et qu'on isole la protéine ainsi obtenue des cellules et du milieu.

**32.** Utilisation du vecteur rétroviral selon les revendications 1 à 21 pour la fabrication d'une préparation pharmaceutique pour la thérapie génique, **caractérisée en ce que** le vecteur est présent sous une forme adéquate pour pouvoir être introduit dans les cellules cibles.

**33.** Utilisation du vecteur rétroviral selon les revendications 18 à 21 pour la fabrication d'une préparation pharmaceutique pour la transfection de cellules souches hématopoïétiques afin d'obtenir une résistance des cellules souches hématopoïétiques en chimiothérapie.

**34.** Préparation pharmaceutique, **caractérisée en ce qu'**elle contient un vecteur selon les revendications 1 à 21 conjointement avec des additifs et/ou substrats pharmaceutiquement acceptables.

FIGUR 1

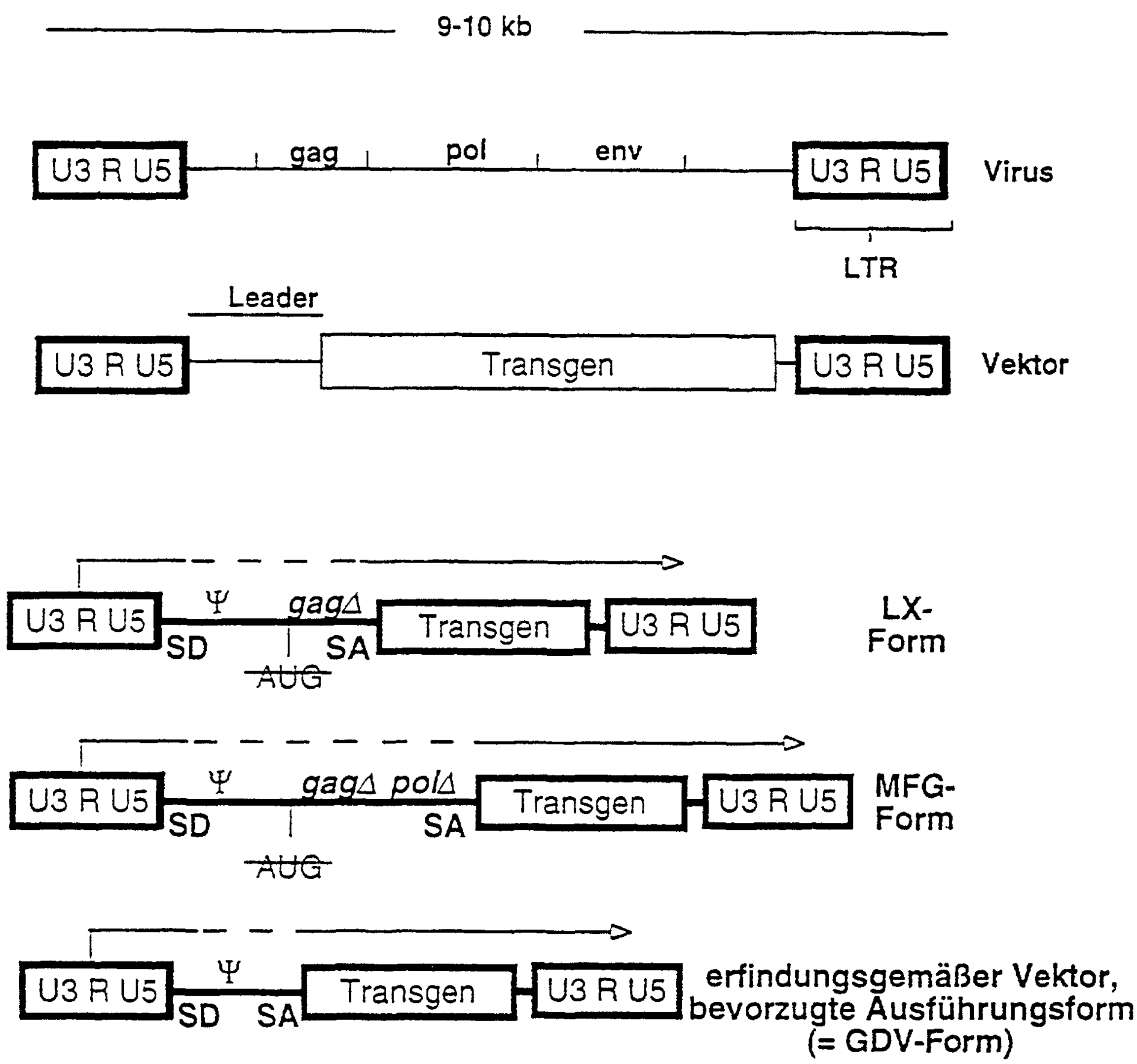
9-10 kb
U3 R U5
gag
pol
env
U3 R U5
Virus
LTR
Leader
U3 R U5
Transgen
U3 R U5
Vektor
U3 R U5
Ψ
gagΔ
SD
SA
AUG
Transgen
U3 R U5
LX-
Form
U3 R U5
Ψ
gagΔ polΔ
SD
SA
AUG
Transgen
U3 R U5
MFG-
Form
U3 R U5
Ψ
SD
SA
Transgen
U3 R U5
erfindungsgemäßer Vektor,
bevorzugte Ausführungsform
(= GDV-Form)

FIGUR 2

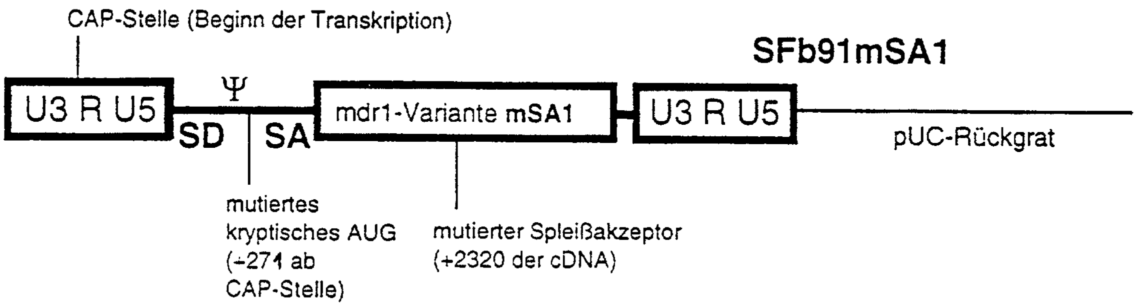
CAP-Stelle (Beginn der Transkription)
SFb91mSA1
U3 R U5
Ψ
SD
SA
mdr1-Variante mSA1
U3 R U5
pUC-Rückgrat
mutiertes kryptisches AUG (+271 ab CAP-Stelle)
mutierter Spleißakzeptor (+2320 der cDNA)

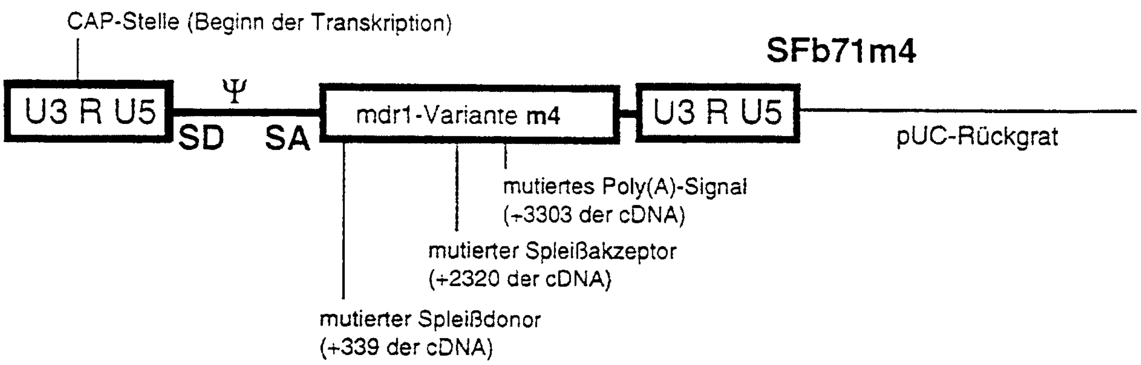
CAP-Stelle (Beginn der Transkription)
SFb71m4
U3 R U5
Ψ
SD
SA
mdr1-Variante m4
U3 R U5
pUC-Rückgrat
mutiertes Poly(A)-Signal (+3303 der cDNA)
mutierter Spleißakzeptor (+2320 der cDNA)
mutierter Spleißdonor (+339 der cDNA)

FIGUR 3

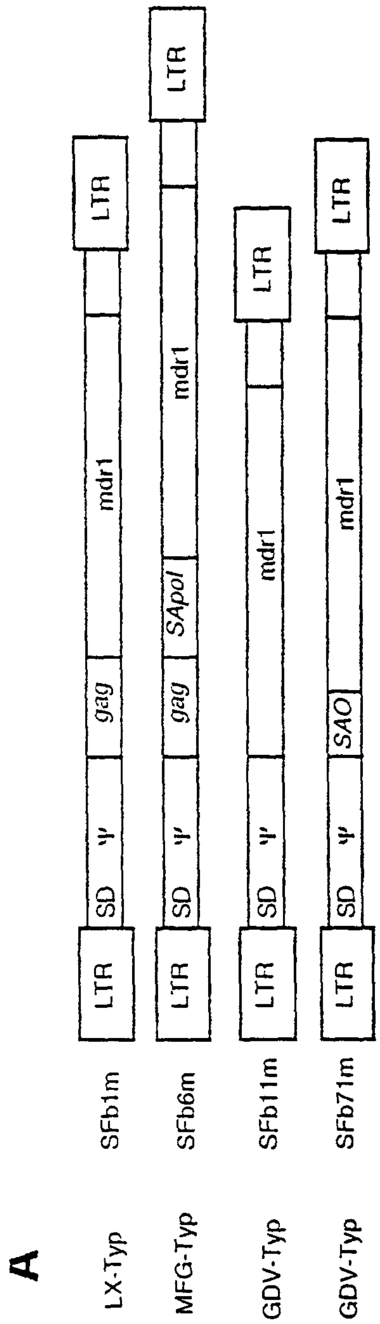
A
LX-Typ
SFb1m
LTR
SD
Ψ
gag
mdr1
LTR
MFG-Typ
SFb6m
LTR
SD
Ψ
gag
SApol
mdr1
LTR
GDV-Typ
SFb11m
LTR
SD
Ψ
mdr1
LTR
GDV-Typ
SFb71m
LTR
SD
Ψ
SAO
mdr1
LTR

FIGUR 3

B

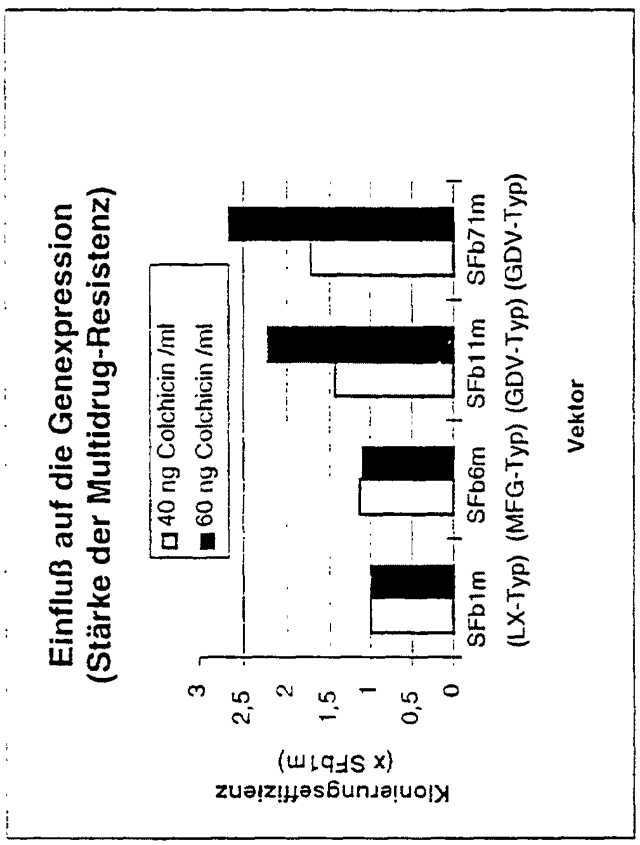
Einfluß auf die Genexpression
(Stärke der Multidrug-Resistenz)
40 ng Colchicin /ml
60 ng Colchicin /ml
Klonierungseffizienz
(x SFb1m)
3
2,5
2
1,5
1
0,5
0
SFb1m
(LX-Typ)
SFb6m
(MFG-Typ)
SFb11m
(GDV-Typ)
SFb71m
(GDV-Typ)
Vektor

FIGUR 4

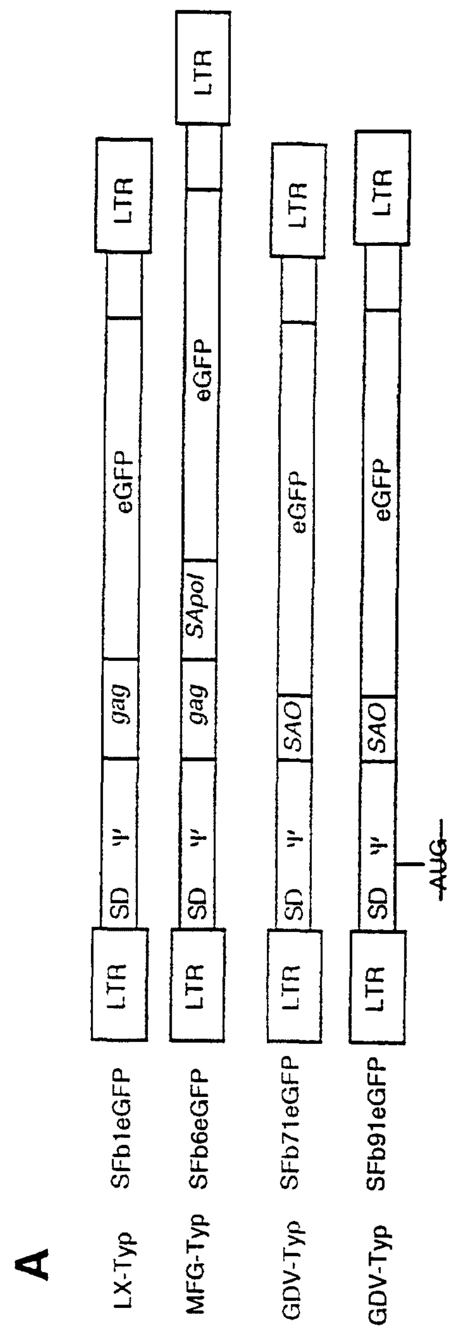
A
LX-Typ
SFb1eGFP
MFG-Typ
SFb6eGFP
GDV-Typ
SFb71eGFP
GDV-Typ
SFb91eGFP
LTR
SD
Ψ
gag
SApol
SAO
eGFP
AUG

FIGUR 4

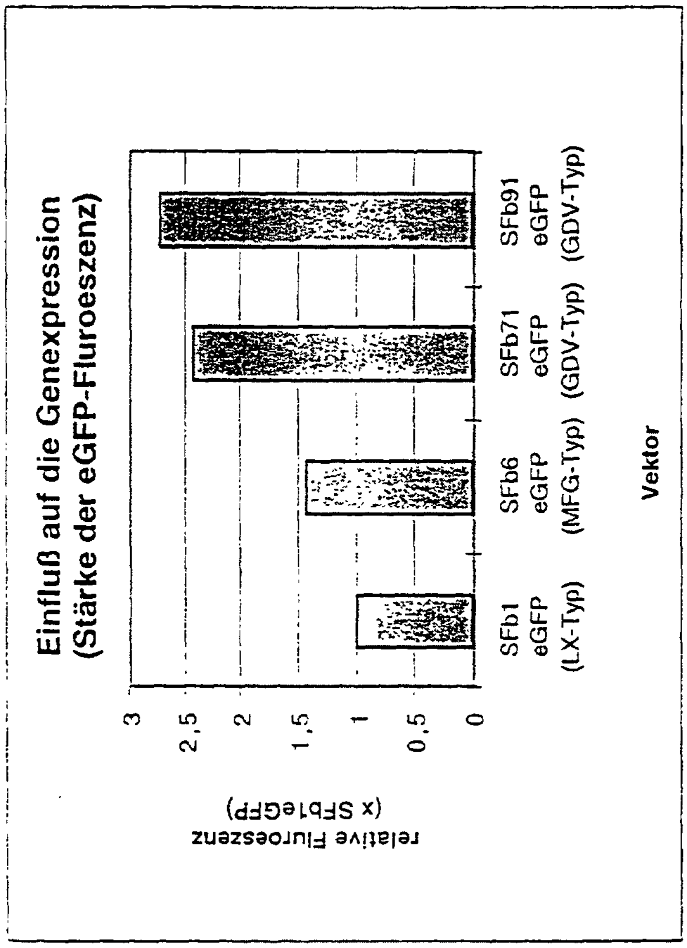
Einfluß auf die Genexpression
(Stärke der eGFP-Fluroeszenz)
relative Fluroeszenz
(x SFb1eGFP)
3
2,5
2
1,5
1
0,5
0
SFb1 eGFP (LX-Typ)
SFb6 eGFP (MFG-Typ)
SFb71 eGFP (GDV-Typ)
SFb91 eGFP (GDV-Typ)
Vektor

B

FIGUR 5

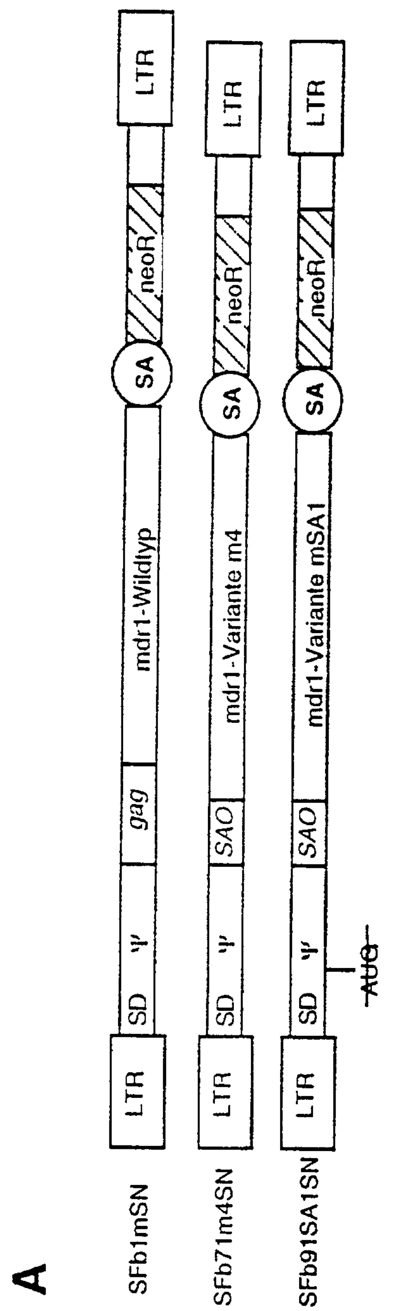
A
SFb1mSN
SFb71m4SN
SFb91SA1SN
LTR
SD
Ψ
gag
SAO
mdr1-Wildtyp
mdr1-Variante m4
mdr1-Variante mSA1
SA
neoR
AUG

FIGUR 5

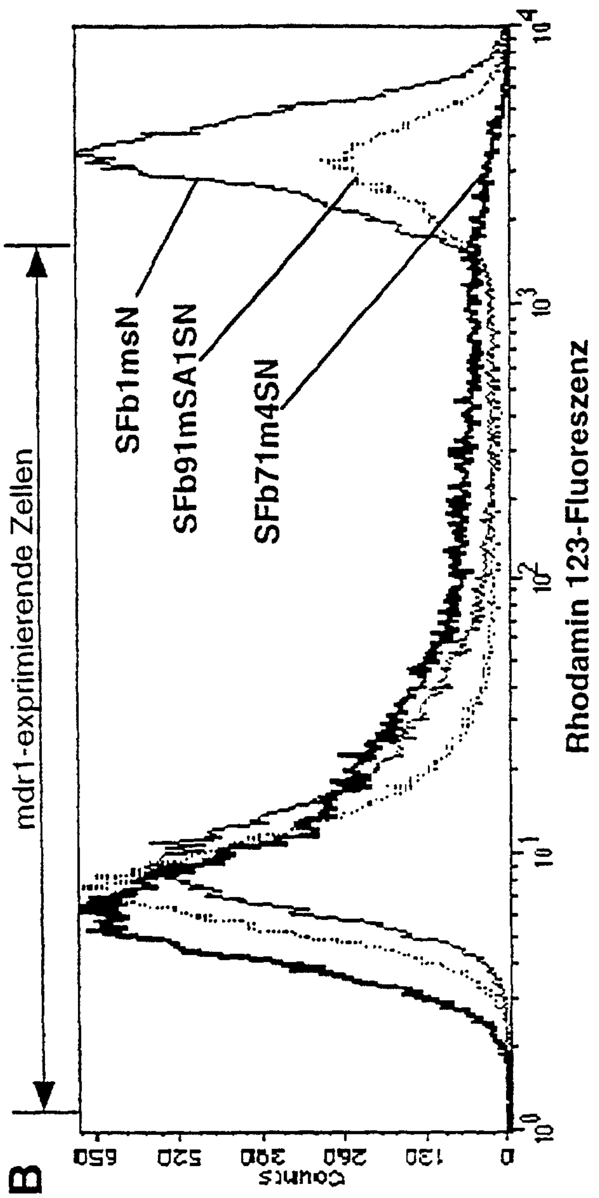
B
mdr1-exprimierende Zellen
SFb1msN
SFb91mSA1SN
SFb71m4SN
Counts
650
520
390
260
130
0
10^0
10^1
10^2
10^3
10^4
Rhodamin 123-Fluoreszenz